# EUROPEAN PATENT SPECIFICATION

(11) **EP 2 711 359 B1**
(45) Date of publication and mention of the grant of the patent: **24.02.2016**
(21) Application number: 13176064.7
(22) Date of filing: 09.12.2011
(51) Int. Cl.: C07D 209/86, H01G 9/20, H01L 51/00, C07D 209/88

(54) **Compounds having hole conducting property, co-adsorbent body comprising same, and dye-sensitized solar cell comprising the co-adsorbent body**
Verbindungen mit Lochleitungseigenschaft, Co-Absorptionskörper damit und farbstoffsensibilisierte Solarzelle mit Co-Absorptionskörper
Composés présentant une propriété conductrice à trous, corps co-adsorbant comprenant ceux-ci et cellule solaire sensibilisée par colorant comprenant le corps co-adsorbant

(30) Priority: 10.12.2010 KR 20100126540
(43) Date of publication of application: 26.03.2014
(62) Divisional of application: 11846378.5
(73) Proprietor: Korea University Research and Business Foundation, Seoul 136-713 (KR)
(72) Inventor: Kim, Hwan-Kyu, 339-763 Sejong-si (KR); Song, Bok-Joo, 305-325 Daejeon (KR); Seo, Kang-Deuk, 306-767 Daejeon (KR); Kang, Min-Soo, 339-761 Sejong-si (KR); Ju, Myung-Jong, 360-050 Chungcheongbuk-do (KR); Song, Hae-Min, 339-803 Sejong-si (KR); Choi In-Taek, 158-095 Seoul (KR); Kim, Sang-Gyun, 361-869 Chungcheongbuk-do (KR); Song, Sang-Hyun, 136-143 Seoul (KR)
(74) Representative: Regimbeau

(56) References cited:
- BOK JOO SONG ET AL: "A Desirable Hole-Conducting Coadsorbent for Highly Efficient Dye-Sensitized Solar Cells through an Organic Redox Cascade Strategy", CHEMISTRY - A EUROPEAN JOURNAL, vol. 17, no. 40, 26 September 2011 (2011-09-26), pages 11115-11121, XP055100570, ISSN: 0947-6539, DOI: 10.1002/chem.201100813
- SRINIVAS K ET AL: "D-pi-A organic dyes with carbazole as donor for dye-sensitized solar cells", SYNTHETIC METALS, ELSEVIER SEQUOIA, LAUSANNE, CH, vol. 161, no. 1-2, 8 December 2010 (2010-12-08), pages 96-105, XP027578932, ISSN: 0379-6779 [retrieved on 2010-12-29]
- CHAO TENG ET AL: "Two Novel Carbazole Dyes for Dye-Sensitized Solar Cells with Open-Circuit Voltages up to 1 V Based on Br - /Br 3 - Electrolytes", ORGANIC LETTERS, vol. 11, no. 23, 3 December 2009 (2009-12-03), pages 5542-5545, XP055101179, ISSN: 1523-7060, DOI: 10.1021/ol9022936

## Description

### BACKGROUND OF THE INVENTION

### 1. Technical Field

The present invention relates to a compound having hole conductivity, a coadsorbent having hole conduction characteristics and including the compound, and a dye-sensitized solar cell including the coadsorbent.

### 2. Background Art

The action principle of a dye-sensitized solar cell can be explained by the mechanism in which, when solar light (visible light) is absorbed in an N-type nanosized semiconductor oxide electrode whose surface is chemically adsorbed with dye molecules, the dye molecules produce electron-hole pairs, the electrons are injected into the conduction band of the semiconductor oxide and transferred to a transparent conductive film through an interface between nanoparticles to generate an electric current, and the holes receive electrons by an oxidation-reduction electrolyte to be reduced again.

In particular, the light conversion efficiency of a dye-sensitized solar cell, unlike other types of solar cells, greatly depends on the characteristics of materials rather than a cell fabrication process. The factors having the greatest influence on the light conversion efficiency of a dye-sensitized solar cell in terms of materials may include a dye, an electrolyte and a transparent metal oxide nanostructure. The light conversion efficiency of a dye-sensitized solar cell greatly depends on the combination of these factors.

Organic dyes have the following advantages: 1) they can easily absorb light because they have high light absorption efficiency (π → π* transition in molecule); 2) they can freely control a light absorption wavelength band because they can easily design various molecular structures; 3) they do not have resource limitations because they do not use metals; and 4) they can be synthesized at a much lower cost than organic metal dyes. In contrast to these advantages, organic dyes have the following disadvantages: 1) they have lower light absorption efficiency than organic metal dyes; 2) the π-π stacking attributable to intermolecular attraction easily occurs because of the characteristics of π-conjugated organic molecules; 3) the lifespan of the excited state thereof after light absorption is short because of the characteristics of organic matter; and 4) they cannot easily absorb the visible light in the entire wavelength range thereof because the width of the wavelength band of an absorption spectrum in a visible light range is not large.

Currently, the maximum efficiency of a dye-sensitized solar cell using an organic metal dye is about 11.2%. Recently, with the rapid advancement of organic dyes, the efficiency of organic dyes has approximated the maximum efficiency thereof However, higher efficiency than that of an organic metal dye has not been reported, and the maximum efficiency of an organic metal dye has become stationary for last several years. Therefore, in order to increase the efficiency of a dye-sensitized solar cell, it is required to develop new dyes, but it is also required to optimize an oxide electrode whose efficiency can be increased by developing a material capable of increasing a conduction band of an N-type semiconductor to increase the open voltage (V_{oc}) thereof.

The molecular structure of a dye plays an important part in a dye-sensitized solar cell (DSSC). The DSSC absorbs light and then begins to separate electric charges at the interface between a dye and a metal oxide. In this case, the performance of a solar cell is determined by the energy level of a dye and the electron transfer process at the interface of a metal oxide.

Meanwhile, the efficiency of the DSSC can be increased by the addition of adducts besides the molecular design of a dye. For example, the efficiency of the DSSC can be increased by the addition of TBP (4-*tert*-butylpyridine) or by using deoxycholic acid (DCA) as a coadsorbent. Specifically, DCA prevents a dye from agglomerating on the surface of a metal oxide to improve the efficiency of injecting electrons into a metal oxide from a dye. Further, the usage of DCA decreases the amount of a dye adsorbed on the surface of a metal oxide, thus providing an effect of improving photocurrent and photovoltage. The maximum value of an open-circuit voltage (V_{OC}) is a difference between the Fermi level of a metal oxide and the oxidation-reduction potential of an oxidation-reduction pair at the time of irradiation a metal oxide using a solar light simulator (AM 1.5G, 100mWcm⁻²). When TBP is added to an electrolyte, the conduction band level of a metal oxide is greatly increased, and the open-circuit voltage (V_{OC}) and fill factor (FF) of the DSSC are improved, thus improving the total efficiency of the DSSC. The improvement of the open-circuit voltage (V_{OC}) means a reduction of a dark current, that is, a reduction of recombination of triiodide ions (I₃⁻) included in the injected electrons and electrolyte.

However, since the above-mentioned conventional method cannot sufficiently prevent the recombination of triiodide ions (I₃⁻) included in the injected electrons, oxidized dye and electrolyte, it is inferred that the DSSC exhibits a low open-circuit voltage (V_{OC}) which is lower than the theoretical value thereof

### SUMMARY OF THE INVENTION

Accordingly, an object of the present invention is to provide a compound having hole conduction characteristics as a novel concept coadsorbent that can be used instead of conventionally-used deoxycholic acid (DCA), wherein a new oxidation-reduction potential that is lower than that of a conventional I₃⁻/I⁻ system is formed to decrease the open voltage loss with a dye, the difference between the Fermi level of an N-type semiconductor conduction band and the oxidation-reduction potential lower than basic potential is greatly increased to increase an open voltage (V_{oc}), the agglomeration of dye particles is prevented to increase the efficiency of injecting electrons into a metal oxide from a dye and thus increase Jsc values, electrical conductivity and ionic conductivity are improved by high hole transporting capacity to increase resistance at the interface of TiO₂/dye/electrolyte and provide higher Jsc values, the compound can be adsorbed on the surface of TiO₂ without the problem of pore filling because the compound has a small molecular weight to allow the holes formed in the dye molecule to be more rapidly transferred to a coadsorbent material, and the recombination of electrons occurring at the interface between a semiconductor layer composed of TiO₂ and an electrolyte including iodine is reduced.

Another object of the present invention is to provide a dye-sensitized solar cell, the photocurrent and photovoltage of which can be improved because it includes the coadsorbent having hole conduction characteristics on a light-absorbing layer.

An aspect of the present invention provides a compound represented by Formula 3: 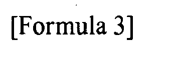 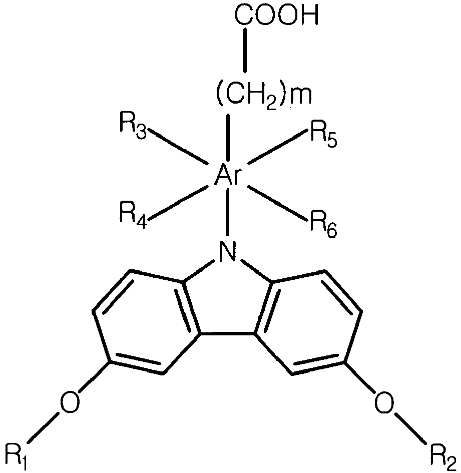 wherein Ar is an aromatic ring of C5 ~ C20 or an aromatic heterocycle of C5~C20, and the aromatic heterocycle includes 1 ~ 3 heteroatoms selected from the group consisting of O, S and N;
R1, R2, R3, R4, R5 and R6 are each independently hydrogen; alkyl of C1 ~ C15 unsubstituted or substituted with alkoxy of C1 ~ C15; alkoxy of C1 ~ C15 unsubstituted or substituted with alkyl of C1 ~ C 15; alkoxy of C1 ~ C 15 unsubstituted or substituted with alkoxy of C1 ~ C15; aryl or heteroaryl of C5 ~ C20 unsubstituted or substituted with a substituent selected from the group consisting of alkyl of C1 ~ C15 unsubstituted or substituted with alkoxy of C1 ~ C15, alkoxy of C1 ~ C15 unsubstituted or substituted with alkyl of C1 ~ C15, and alkoxy of C1 ~ C15 unsubstituted or substituted with alkoxy of C1 ~ C15; or arylalkyl or heteroarylalkyl of C6 ~ C22 unsubstituted or substituted with a substituent selected from the group consisting of alkyl of C1 ~ C15 unsubstituted or substituted with alkoxy of C1 ~ C15, alkoxy of C1 ~ C15 unsubstituted or substituted with alkyl of C1 ~ C15 and alkoxy of C1 ~ C 15 unsubstituted or substituted with alkoxy of C1 ~ C 15;
m is an integer of 0 ~ 5; and
one or more of R3, R4, R5 and R6 are nonexistent when the Ar is an aromatic heterocycle,

Another aspect of the present invention provides a coadsorbent having hole conduction characteristics, including the compound represented by Formula 3.

Still another aspect of the present invention provides a dye-sensitized solar cell including a light absorbing layer including the coadsorbent having hole conduction characteristics.

According to the compound having hole conduction characteristics of the present invention, a new oxidation-reduction potential that is lower than that of a conventional I₃⁻/I⁻ system is formed to decrease the open voltage loss with a dye, and the difference between the Fermi level of an N-type semiconductor conduction band and the oxidation-reduction potential that is lower than the basic potential is greatly increased to increase an open voltage (V_{oc}).

Further, according to the compound having hole conduction characteristics of the present invention, the agglomeration of dye particles is prevented to increase the efficiency of injecting electrons into a metal oxide from a dye and thus increase Jsc values, electrical conductivity and ionic conductivity are improved by high hole transporting capacity to increase resistance at the interface of TiO₂/dye/electrolyte and provide higher Jsc values, the compound can be adsorbed on the surface of TiO₂ without the problem of pore filling because the compound has a small molecular weight to allow the holes formed in the dye molecule to be more rapidly transferred to a coadsorbent material, and the recombination of electrons occurring at the interface between a semiconductor layer composed of TiO₂ and an electrolyte including iodine is reduced, thereby improving the photocurrent and photovoltage of a dye-sensitized solar cell.

Further, the compound having hole conduction characteristics according to the present invention can be very usefully used to improve the efficiency of a solar cell because its price is low.

### BRIEF DESCRIPTION OF THE DRAWINGS

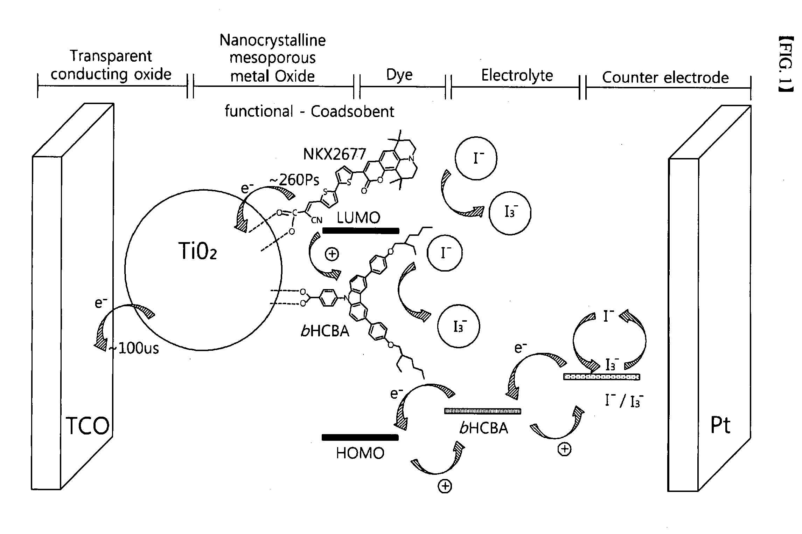

FIG. 1 is a schematic view showing the function of a coadsorbent having hole conduction characteristics in a dye-sensitized solar cell.
FIG. 2 is a graph showing the UV-vis absorption spectra measured by adsorbing dye solutions prepared in an Example and Comparative Examples on a TiO₂ film.
FIG. 3 is a graph showing the current-voltage curves of dye-sensitized solar cells manufactured in an Example and Comparative Examples.
FIG. 4 is a graph showing the incident photon to current conversion efficiencies (IPCEs) of dye-sensitized solar cells manufactured in an Example and Comparative Examples.
FIG. 5 is a graph showing the Nyquist plat obtained by measuring AC impedances under a condition of 1 sun (100 mW/cm²) in order to measure the charge transfer resistance in a dye-sensitized solar cell.
FIG. 6 show an equivalent circuit for obtaining internal resistance in dye-sensitized solar cells manufactured in an Example and Comparative Examples.
FIG. 7 is a graph showing the current-voltage curves of dye-sensitized solar cells manufactured in an Example and Comparative Examples.
FIG. 8 is a graph showing the incident photon to current conversion efficiencies (IPCEs) of dye-sensitized solar cells manufactured in an Example and Comparative Examples.

### DESCRIPTION OF THE PREFERRED EMBODIMENTS

The present invention is directed to a compound represented by Formula 3.

The compound represented by Formula 3 has excellent hole conduction characteristics.

In the Formula 3, the alkyl of C1 ~ C15 included in each of the substituents may be methyl, ethyl, propyl, butyl, pentyl, hexyl, heptyl, octyl or the like, and the alkoxy of C1 ~ C15 may be methoxy, ethoxy, propoxy, butoxy, pentoxy, hexoxy, heptoxy or the like.

For example, the alkyl of C1 ∼ C15 substituted with alkoxy of C1 ∼ C15 may be butoxymethyl, butoxyethyl, hexoxymethyl, heptoxymethyl or the like, the alkoxy of C1 ~ C15 substituted with alkyl of C1 ~ C15 may be 2-ethylheptyloxy, 3-ethylheptyloxy, 2-methylbutyloxy, 2-ethylpentyloxy, 3-ethylpentyloxy or the like, and the alkoxy of C1 ~ C15 substituted with alkoxy of C1 ~ C15 may be 3-methoxypentoxy, 3-ethoxypentoxy, 3-propoxypentoxy, 2-methoxyhexoxy, 2-ethoxyhexoxy, 2-propoxyhexoxy or the like.

Further, examples of the aryl or heteroaryl included in the aryl or heteroaryl of C5 ~ C20 and arylalkyl or heteroarylalkyl of C6 ~ C22 may inc lude, but are not limited to, phenyl, naphthyl, thiophenyl, anthracyl, imidazole, pyridine, oxazole, thiazole, quinoline, EDOT (3,4-ethylenedioxythiophene) and the like.

Examples of the Ar may include, but are not limited to, phenyl, naphthalene, anthracene, imidazole, pyridine, oxazole, thiazole, quinoline, EDOT (3,4-ethylenedioxythiophene) and the like.

The alkyl included in each of the substituents may be straight chain alkyl or branch chain alkyl.

The compound having hole conduction characteristics according to the present invention can be used to prepare a novel concept coadsorbent that can be used instead of conventionally-used deoxycholic acid (DCA) in a dye-sensitize solar cell, and is characterized in that it has excellent function of preventing the agglomeration of dye, and in that it can penetrate a TiO₂ interface in a small amount to minimize the problem of pore filling.

Hereinafter, specific examples of the novel compounds of the present invention and methods of preparing the same will be described in detail.

A specific example of the compounds represented by the Formula 3 above is as follows:
4-(3,6-bis(2-ethylhexyoxy)-9H-carbazole-9-yl)benzoic acid.

Each of the compounds represented by Formula 3 above can be usefully used as a coadsorbent. Therefore, the present invention provides a coadsorbent having hole conduction characteristics and including any one of the compounds represented by Formula 3 above.

Further, the present invention provides a dye-sensitized solar cell including a light absorbing layer including the coadsorbent having hole conduction characteristics.

According to the dye-sensitized solar cell including a light absorbing layer including the coadsorbent having hole conduction characteristics, a dye can be rapidly recharged because hole conductivity is improved by the coadsorbent, the π-πstacking between dye particles can be prevented, and the recombination of electrons having passed through a TiO₂ layer with an electrolyte or dye can be prevented to give high JSC values and high V_{OC} values.

The dye-sensitized solar cell of the present invention may includes the following constituents: a first electrode including a transparent conductive substrate; a light absorbing layer formed on one side of the first electrode; a second electrode facing the first electrode provided with the light absorbing layer; and an electrolyte disposed between the first electrode and the second electrode.

The components constituting the dye-sensitized solar cell will be illustrated as follows.

The first electrode including a transparent conductive substrate may be a light transmissive electrode made of one or more selected from the group consisting of indium-tin oxide, fluorine-tin oxide, ZnO- Ga₂O₃, ZnO-Al₂O₃ and tin oxides, and formed on a glass substrate or a plastic substrate.

The light absorbing layer includes semiconductor particles, a dye, a compound having hole conduction characteristics, and the like. The semiconductor particles may be formed of oxide nanoparticles, such as titanium dioxide (TiO₂) nanoparticles, tin dioxide (SnO₂) nanoparticles, zinc oxide (ZnO) nanoparticles and the like. The dye adsorbed on the semiconductor particles can be used without limitation as long as it can absorb visible light, can be strongly chemically-bonded with oxide nanoparticles and has thermal and optical stability. A typical example of the dye may be a ruthenium-based organic metal compound. The coadsorbent having hole conduction characteristics fills the holes formed in the dye having absorbed light to donate electrons and is then converted into holes, and these holes are filled with an electrolyte again.

The second electrode may be the same as the first electrode, and may be a light transmissive electrode provided thereon with an electrical collection layer made of platinum.

Hereinafter, the present invention will be described in more detail with reference to the following Examples. However, these Examples are set forth to more clearly illustrate the present invention, and the scope of the present invention is not limited thereto. The scope of the present invention will be defined by the technical ideas of claims.

### Examples 1 to 20 and Comparative Examples A to D

### Used reagents

As reagents, tetrahydrofuran, acetonitrile, sulfuric acid, toluene, methanol, acetic acid, ethanol, acetone, ethyl acetate, hydrochloric acid, hexane and dichloromethane, manufactured by Dong Yang Chemical Co., Ltd., were used.

Further, as other reagents, 2-ethoxyethanol, 4,4'-dibromobiphenyl, CuCN (copper cyanide), dimethyl sulfoxide, 1,2-dichlorobenzene, dimethyl sulfate, N,N-dimethylformamide, 2-isopropoxy-4,4,5,5-tetramethyl-1,3,2-dioxaborolane, *N-*bromosuccinimide, sulfamic acid, sodium chlorite, phosphoryl chloride, cuprous chloride (I), 1,10-phenanthroline, zinc (Zn), potassium hydroxide, potassium carbonate, iodine, orthoperiodic acid, methyl iodide, methyl 4-bromobenzoate, Cu-bronze, 19-crown-6, N-butyllithium, sodium carbonate, sodium hydroxide, sodium bicarbonate, sodium sulfate, 9H-fluorene, 4-iodophenol, 3-(bromomethyl)hexane, carbazole, 4-fluorobenzonitrile, potassium-tert-butoxide, 3,5-dibromobenzonitrile, 1,2-dimethyl-3-propylimidazolium iodine, lithium iodide (LiI), I₂, 18-crown-6, K₂CO₃-tetrabutylammonium hexafluorophosphate, 1,3-dimethyl-5-nitrobenzene, aluminum trichloride, 1-chlorohexane, and 2-ethyl-1-hexanol, manufactured by Aldrich Corporation, were used. These reagents were used without additional purification.

### Method of observing synthesized compound,

The structures of all novel compounds were observed by ¹H-NMR, ¹³C-NMR and FT-IR. ¹H-NMR was recorded using a Varian 300 spectrometer, and all chemical mobility was recorded by a unit of ppm using tetamethylsilane which is an internal standard material. IR spectra were measured by KBr pellets using a Perkin-Elmer spectrometer.

### Comparative Example 1: Synthesis of 2-(4-(bis(4-(2-ethylhexyloxy)phenyl)amino)phenyl) acetic acid

### 1-1: Synthesis of 1-(2-ethylhexyloxy)-4-iodobenzene

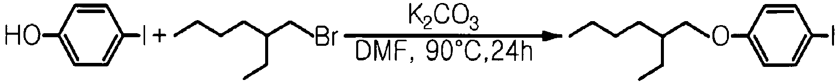

4-iodophenol (15 g, 68.18 mmol), K₂CO₃ (24.5 g, 177.26 mmol) and DMF (70 mL) were put into a 250 mL round flask, and then refluxed and stirred for 2 hours to prepare a first mixture. Then, 2-ethylhexylbromide (17.12 g, 88.63 mmol) was added to the first mixture using an injector, and then refluxed and stirred for 24 hours to prepare a second mixture. When the reaction of the second mixture was finished, the reaction mixture was cooled to room temperature, acid-treated with 70 mL of an aqueous 2 M HCl solution, extracted with methylene chloride, and then washed with distilled water several times to form an organic layer. The organic layer was dried by magnesium sulfate (MgSO₄), a solvent was removed from the dried organic layer under reduced pressure, and a reaction product was separated by column chromatography (silica, CH₂Cl₂: hexane = 3:2). The yield of the reaction product was 93%.
¹H NMR(CDCl₃. ppm): δ7.55-7.52(d, 2H, Ar-H), 6.69-6.66(d, 2H, Ar-H), 3.80-3.79(d, 2H, CH₂-O), 1.73-1.67(m, 1H,(CH₂)₃-H), 1.53-1.28(m, 8H, -CH₂), 0.97-0.88(t, 6H, -CH₃).

### 1-2: Synthesis of 4-di(4-(2-ethylhexyloxy)phenyl)aminobenzene

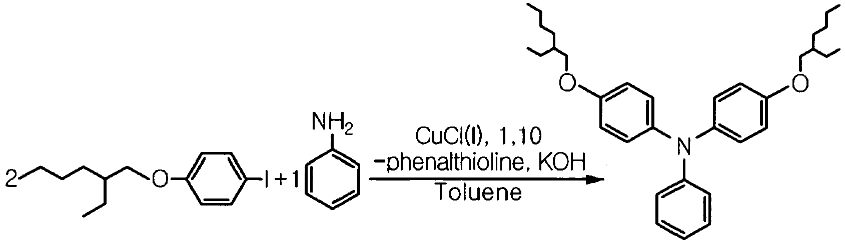

4-(2-ethylhexyloxy)benzyl iodide (19.03 g, 59.06 mmol), aniline (2.5 g, 26.84 mmol), copper chloride (0.27 g, 2.68 mmol), 1,10-phenanthroline (0.48 g, 2.68 mmol), KOH (9.04g, 161.07 mmol) were put into a 250 mL round flask to prepare a first mixture. Then, 20 mL of purified toluene was added to the first mixture using an injector under a nitrogen atmosphere, and then refluxed and stirred at 125°C for 24 hours to prepare a second mixture. When the reaction of the second mixture was finished, the reaction mixture was cooled to room temperature, extracted with toluene, and then washed with distilled water several times to form an organic layer. The organic layer was dried by magnesium sulfate (MgSO₄), a solvent was removed from the dried organic layer under reduced pressure, and a reaction product was separated by column chromatography (silica, CH₂Cl₂: hexane = 2:3). The yield of the reaction product was 80%.
¹H NMR(CDCl₃. ppm): δ7.23-7.21(t, 1H, Ar-H), 6.85-6.81(t, 2H, Ar-H), 6.79-6.75(d, 4H, Ar-H), 6.63-6.61(d, 2H, Ar-H), 6.55-6.52(d, 4H, Ar-H), 3.80-3.79(d, 4H, CH₂-O), 1.73-1.67(m, 2H,(CH₂)₃-H), 1.53-1.28(m, 16H, -CH₂), 0.97-0.88(t, 12H,-CH₃).

### 1-3: Synthesis of 4-di(4-(2-ethylhexyloxy)phenyl)aminobenzaldehyde

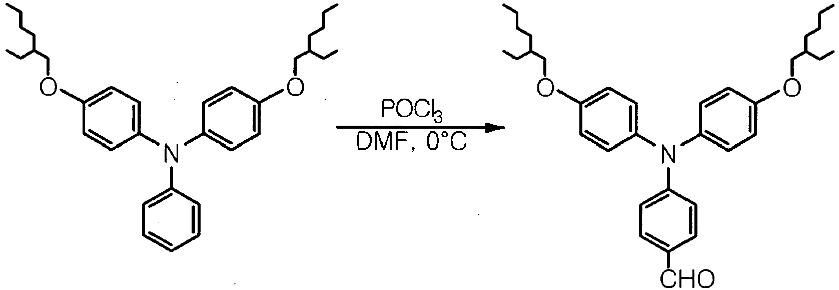

A 250 mL Schlenk flask was provided with a dropping funnel filled with 4-(2-ethylhexyloxy)-N-(4-(2-ethylhexyloxy)phenyl)-N-phenylbenzeneamine (5g, 20.38 mmol), and was then dried in vacuum. Subsequently, POCl₃ (3.28 g, 21.04 mmol) and DMF (30 mL) were put into the flask using an injector under a nitrogen reflux. Thereafter, 20 mL of DMF was additionally put into the dropping funnel, and then the mixture of DMF and 4-(2-ethylhexyloxy)-N-(4-(2-ethylhexyloxy)phenyl)-N-phenylbenzeneamine was slowly dripped therein at 0°C. After the dripping of the mixture was finished, refluxing and stirring were performed at 90 °C for 24 hours. When the reaction of the mixture was finished, the reaction mixture was cooled to room temperature, extracted with MC, and then washed with distilled water to form an organic layer. The organic layer was dried by magnesium sulfate (MgSO₄), a solvent was removed from the dried organic layer under reduced pressure, and a reaction product was separated by column chromatography (silica, CH₂Cl₂: hexane = 2:3). The yield of the reaction product was 80%.
¹ H NMR(CDCl₃. ppm): δ9.9(s, 1H, H-C=O), 7.09-7.07(d, 2H, Ar-H), 6.85-6.82(d, 2H, Ar-H), 6.77-6.74(d, 4H, Ar-H), 6.54-6.52(d, 4H, Ar-H), 3.80-3.79(d, 4H, CH₂-O), 1.73-1.67(m, 2H,(CH₂)₃-H), 1.53-1.28(m, 16H, -CH₂), 0.97-0.88(t, 12H,-CH₃).

### 1-4: Synthesis of 4-di(4-(2-ethylhexyloxy)phenyl)aminobenzoic acid

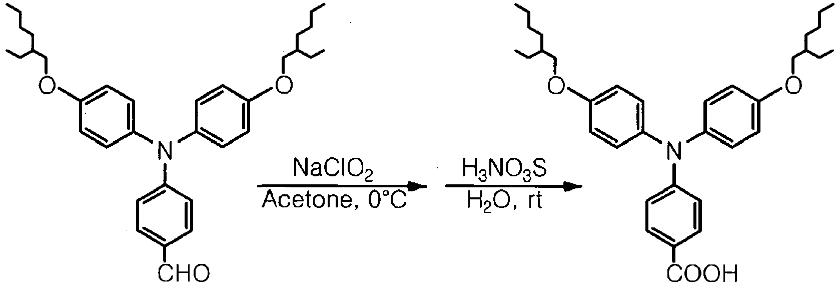

4-di(4-(2-ethylhexyloxyphenyl)aminobenzaldehyde and sodium chlorite (NaClO₂) were mixed with acetone, and then slowly stirred at 0 °C to prepare a first mixed solution. Subsequently, sulfamic acid and water were added to the first mixed solution, and simultaneously stirred at room temperature for 12 hours to prepare a second mixed solution. Then, the second mixed solution was extracted with water and ethyl acetate to obtain an organic layer. The organic layer was dried by magnesium sulfate (MgSO₄), a solvent was removed from the dried organic layer under reduced pressure, and a reaction product was separated by column chromatography (silica, CH₂Cl₂). The yield of the reaction product was 80%.
¹H NMR(CDCl₃. ppm): δ12.7(s, 1H, H-O-C=O), δ7.09-7.07(d, 2H, Ar-H), 6.85-6.82(d, 2H, Ar-H), 6.77-6.74(d, 4H, Ar-H), 6.54-6.52(d, 4H, Ar-H), 4.61(s, 2H, CH₂), 3.80-3.79(d, 4H, CH₂-O), 1.73-1.67(m, 2H,(CH₂)₃-H), 1.53-1.28(m, 16H,-CH₂), 0.97-0.88(t, 12H, -CH₃).

### Comparative Example 2: Synthesis of 4-(bis(9,9-dimethyl-9H-fluorene-2-yl)amino)benzoic acid

### 2-1: Synthesis of 2-iodofluorene

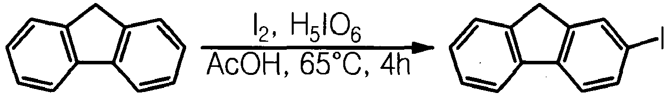

Fluorene (30.0 g, 180 mmol) was dissolved in a boiling solvent (acetic acid: water: sulfuric acid / 100:20:3(v/v/v)) to prepare a first mixed solution, and then periodic acid dehydrate (8.0 g, 45 mmol) and iodine (23.0 g, 91.0 mmol) were added to the first mixed solution to prepare a second mixed solution. Then, the second mixed solution was stirred at 65°C for 4 hours to obtain a precipitate. The precipitate was filtered and then washed with a 2N aqueous sodium carbonate solution and water to obtain crystals. The crystals were recrystallized by hexane. The yield of the product was 72%.
¹H NMR(300 MHz, CDCl₃) δ(TMS, ppm): 3.81(2H, s, -CH2), 7.31(2H, m, Ar-H), 7.44(2H, m, Ar-H), 7.66(1H, d, Ar-H), 7.73(1H, d, Ar-H), 7.85(1H, s, Ar-H).

### 2-2: Synthesis of 9,9-dimethyl-2-iodofluorene

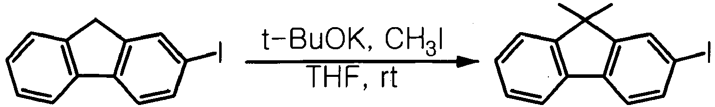

Potassium tert-butoxide (21.8 g, 0.19 mol) was added to cooled anhydrous tetrahydrofuran, in which 2-iodofluorene (25.0 g, 85.6 mmol) was dissolved, and then stirred at room temperature for 1.5 hours to prepare a first mixed solution. Subsequently, methyl iodide (28.2 g, 0.19 mol) was added to the first mixed solution to prepare a second mixed solution, and then the second mixed solution was stirred for 2 hours to obtain potassium iodide. Subsequently, the obtained potassium iodide was filtered, a solvent was removed under reduced pressure, and a reaction product was separated by silica column chromatography (hexane). The yield of the reaction product was 70%.
¹H NMR(300 MHz, CDCl₃) δ(TMS, ppm): 1.47(6H, s, -CH₃), 7.31(2H, m, Ar-H)), 7.45(2H, m, Ar-H), 7.66(1H, d, Ar-H), 7.73(1H, d, Ar-H), 7.85(1H, s, Ar-H).

### 2-3: Synthesis of methyl 4-(bis(9,9-dimethyl-9H-fluorene-2-yl)amino)benzoate

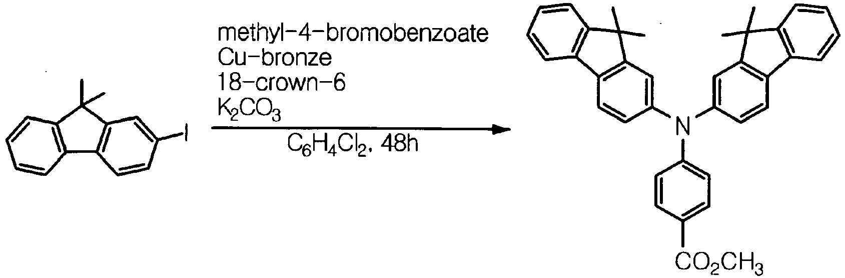

The obtained 9,9-dimethyl-2-iodofluorene (5.00 g, 15.6 mmol), methyl-4-aminobenzate (1.00 g, 6.62 mol), a copper-tin alloy (0.12 g, 0.66 mmol), 18-crown-6 (0.180 g, 0.68 mmol) and potassium carbonate (4.1 g, 29.6 mmol) were dissolved in 1,2-dichlorobenzene, and then refluxed and stirred for 2 days to prepare a mixed solution. After the reaction of the mixed solution was finished, the mixed solution was extracted with dichloromethane and water several times to form an organic layer. The organic layer was dried by magnesium sulfate (MgSO₄) and then filtered, a solvent was removed from the filtered organic layer under reduced pressure, and a reaction product was separated by silica column chromatography (dichloromethane:hexane = 2:1). The yield of the reaction product was 59%.
¹H NMR(300 MHz, CDCl₃) δ(TMS, ppm): 1.43(12H, s, -CH₃), 3.89(3H, s,-CH₃), 7.31(4H, m, Ar-H)), 7.45(2H, m, Ar-H), 7.66(6H, m, Ar-H), 7.73(4H, d, Ar-H), 7.85(2H, d, Ar-H).

### 2-4: Synthesis of 4-(bis(9,9-dimethyl-9H-fluorene-2-yl)amino)benzoic acid

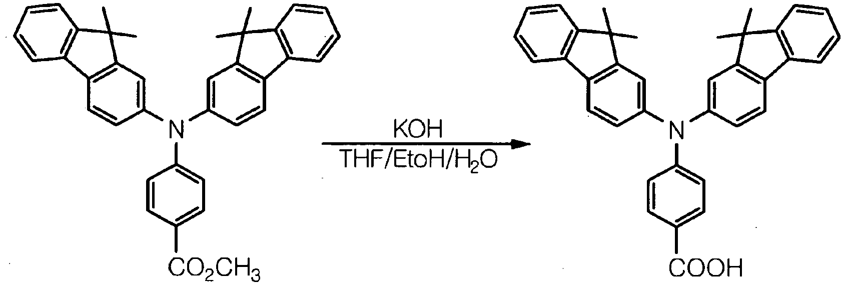

The obtained methyl-4-(bis(9,9-dimethyl-9H-fluorene-2-yl)amino)benzoate (300 mg, 0.58 mmol), KOH (330 mg, 5.88 mmol), THF-ethanol (1:1) 100 mL, distilled water 30 mL were put into a 250 mL flask, and then refluxed and stirred for 1 day to prepare a mixed solution. When the reaction of the mixed solution was finished, the mixed solution was cooled to room temperature, and then concentrated hydrochloric acid was added to the mixed solution to adjust the pH of the mixed solution to 2. This mixed solution was extracted with dichloromethane (CH₂Cl₂) to form an organic layer. The organic layer was washed with an aqueous sodium bicarbonate solution and water several times, and then dried by anhydrous magnesium sulfate (MgSO₄). Then, a solvent was removed from the dried organic layer under reduced pressure, and a reaction product was separated by rapid column chromatography (silica, CH₂Cl₂:EA = 3:2). The separated reaction product was washed with cold ethanol and then dried in vacuum. The yield of the reaction product was 80%.
¹H NMR(300 MHz, CDCl₃) δ(TMS, ppm): 1.43(12H, s, -CH₃), 7.31(4H, m, Ar-H)), 7.45(2H, m, Ar-H), 7.66(6H, m, Ar-H), 7.73(4H, d, Ar-H), 7.85(2H, d, Ar-H).

### Comparative Example 3: Synthesis of 4-(3,6-bis(4-(2-ethylhexyloxy)phenyl)-9H-carbazole-9-yl)benzoic acid

### 3-1: Synthesis of 1-bromo-4-(2-ethylhexyloxy)benzene.

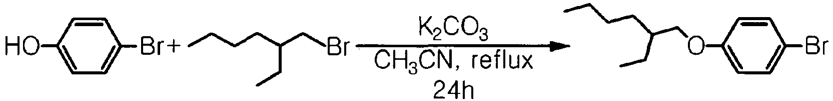

4-bromophenol (5 g, 28.8 mmol) and K₂CO₃ (9.99 g, 43.2 mmol) were mixed with acetonitrile (30 mL) and then stirred at room temperature for 1 hour to prepare a first mixture. Then, 3-(bromomethyl)heptane (6.67 g, 34.6 mmol) was slowly dripped into the first mixture and then stirred at room temperature for 23 hours to prepare a second mixture. After the reaction of the second mixture, the reaction mixture was extracted with water and ethyl acetate and dried by magnesium sulfate (MgSO₄), and then a reaction product was separated by column chromatography (*n*-hexane/DCM = 2/1). The yield of the reaction product was 67% (5.52 g).
¹HNMR(300 MHz,CDCl₃)δ7.55-7.52(d, 2H, Ar-H), 6.69-6.66(d, 2H, Ar-H), 3.80-3.79(d, 2H, CH₂-O), 1.73-1.67(m,1H,(CH₂)₃-H),1.53-1.28(m,8H,-CH₂),0.97-0.88(t,6H,-CH₃)

### 3-2: Synthesis of 2-(4-(2-ethylhexyloxy)phenyl)-4,4,5,5-tetramethyl-1,3,2-dioxaborolane

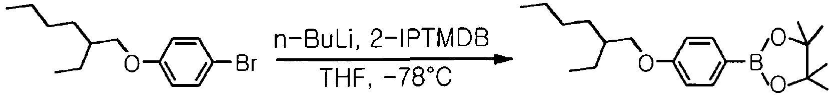

The obtained 1-bromo-4-(2-ethylhexyloxy)benzene (5.52 g, 19.4 mmol) was mixed with THF (150 mL) to prepare a first mixture, and then *n*-BuLi (1.61 g, 25.2 mmol, 2.5 min, *n*-hexane) was dripped into the first mixture at -78°C and stirred for 1 hour to prepare a second mixture. Then, 2-isopropoxy-4,4,5,5-tetramethyl-1,3,2-dioxaborolane (4.32 g, 23.2 mmol) was dripped into the second mixture, stirred for 1 hour and then further stirred at room temperature for 12 hours to prepare a third mixture. After the reaction of the third mixture, a solvent was removed, the reaction mixture was extracted with water and ethyl acetate and dried by magnesium sulfate (MgSO₄), and then a reaction product was separated by column chromatography (*n-*hexane/DCM = 3/1). The yield of the reaction product was 92% (5.9 g).
¹HNMR(300 MHz,CDCl₃)δ7.55-7.52(d, 2H, Ar-H), 6.69-6.66(d, 2H, Ar-H), 3.80-3.79(d, 2H, CH₂-O), 1.73-1.67(m,1H,(CH₂)₃-H),1.53-1.28(m,8H,-CH₂),1.28-1.32(s,12H,-CH₃)1.53-1.28(m,5H,-CH₂),0.97-0.88(t,6H,-CH₃)

### 3-3: Synthesis of 4-(9H-carbazole-9-yl)benzonitrile

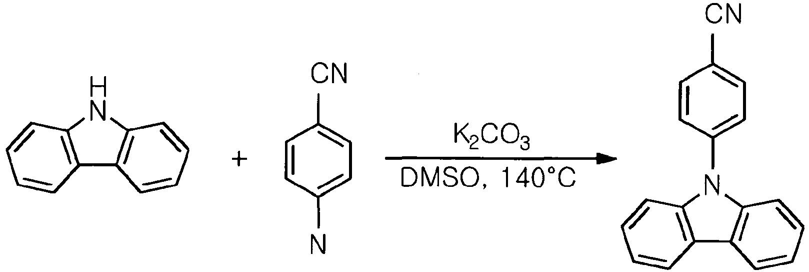

9H-carbazole (3 g, 17.94 mmol) and K₂CO₃ (8.22 g, 26.91 mmol) were mixed with DMSO (30 mL) and then stirred at room temperature for 1 hour to prepare a first mixture. Then, 4-fluorobenzonitrile (2.61 g, 21.5 mmol) was dripped into the first mixture and then stirred at 150°C for 12 hours to prepare a second mixture. After the reaction of the second mixture, a solvent was removed, the reaction mixture was extracted with water and ethyl acetate and dried by magnesium sulfate (MgSO₄), and then a reaction product was separated by column chromatography (*n*-hexane/EtOAc = 2/1). The yield of the reaction product was 94% (4.5 g).
¹HNMR(300 MHz,CDCl₃) 8.13(d, Ar-H, 2H), 7.44(d, Ar-H, 2H), 7.38(m, Ar-H, 2H), 7.32(d, Ar-H, 2H), 7.27(m, Ar-H, 2H), 7.10(d, Ar-H, 2H)

### 3-4: Synthesis of 4-(3,6-dibromo-9H-carbazole-9-yl)benzonitrile

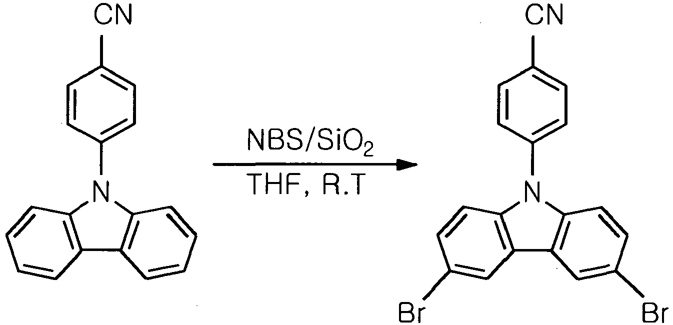

4-(9H-carbazole-9-yl)benzonitrile (4.5 g, 16.8 mmol) was dissolved in THF (50 mL), and then *N*-bromosuccinimide (5.9 g, 33.9 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 90% (6.5 g).
¹HNMR(300 MHz,CDCl₃)δ7.01-7.03(d,-Ar,4H),7.48-7.51(d,-Ar,2H), 8.27(s,-Ar,2H)

### 3-5: Synthesis of 4-(3,6-bis(4-(2-ethylhexyloxy)phenyl)-9H-carbazole-9-yl)benzonitrile

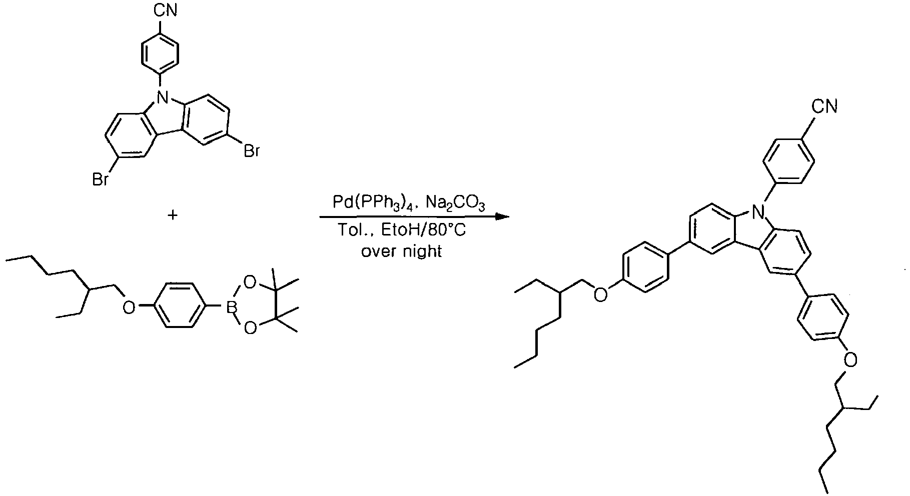

4-(3,6-dibromo-9H-carbazole-9-yl)benzonitrile (3.0 g, 7.04 mmol), 2-(4-(2-ethylhexyloxy)phenyl)-4,4,5,5-tetramethyl-1,3,2-dioxaborolane (4.68 g, 14.08 mmol), sodium carbonate (1.87 g, 10.56 mmol) and tetrakis(triphenylphosphine) palladium (Pd(PPh₃)₄) (0.68 g,0.35 mmol) were dissolved in 100 mL of toluene/tetrahydrofuran/H₂O/ethanol (3:1:1:1(v/v/v/v)) under a nitrogen atmosphere, and then refluxed and stirred at 80°C for 12 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 3/1). The yield of the reaction product was 60% (2.9 g).
¹HNMR(300 MHz,CDCl₃)δ7.55-7.52(d, 2H, Ar-H), 6.69-6.66(d, 2H, Ar-H), 3.80-3.79(d, 2H, CH₂-O),1.73-1.67(m,1H,(CH₂)₃-H),1.53-1.28(m,8H,-CH₂),0.97-0.88(t,6H,-CH₃),δ 7.01-7.03(d, -Ar, 4H), 7.48-7.51(d, -Ar, 2H), 7.63-7.65(m, -Ar, 6H), 8.27(s, -Ar, 2H).

### 3-6: Synthesis of 4-(3,6-bis(4-(2-ethylhexyloxy)phenyl)-9H-carbazole-9-yl)benzoic acid

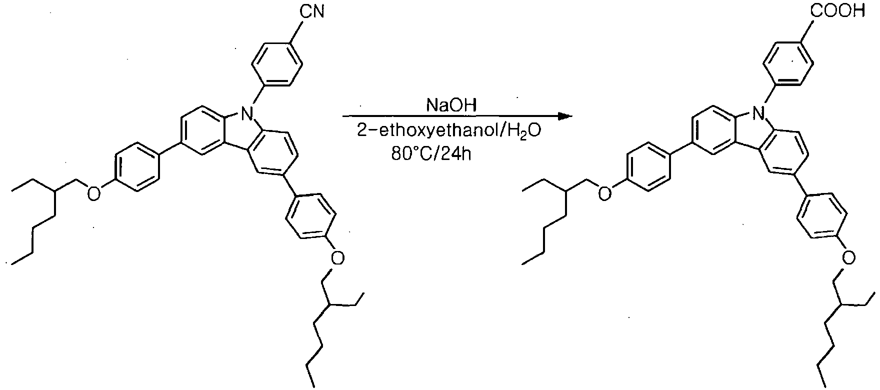

4-(3,6-bis(4-(2-ethylhexyloxy)phenyl)-9H-carbazole-9-yl)benzonitrile (2.9 g, 4.28 mmol) and sodium hydroxide (0.51 g, 12.85 mmol) were dissolved in 20 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO ₄), and then a reaction product was separated by column chromatography (*n-*hexane/EtOAc = 2/1). The yield of the reaction product was 94% (4.5 g).
¹HNMR(300 MHz,CDCl₃)δ7.55-7.52(d, 2H, -Ar), 6.69-6.66(d, 2H, -Ar), 3.80-3.79(d, 2H, CH₂-O),1.73-1.67(m,1H,(CH₂)₃-H),1.53-1.28(m,8H,-CH₂),0.97-0.88(t,6H,-CH₃),δ 7.01-7.03(d, -Ar, 4H), 7.48-7.51(d, -Ar, 2H), 7.63-7.65(m, -Ar, 6H), 8.27(s, -Ar, 2H),(s, OH, H)

### Comparative Example 4: Synthesis of 4'-(3,6-bis(4-(2-ethylhexyloxy)phenyl)-9H-carbazole-9-yl)biphenyl-4-carboxylic acid

### 4-1: Synthesis of 1-bromo-4-(2-ethylhexyloxy)benzene

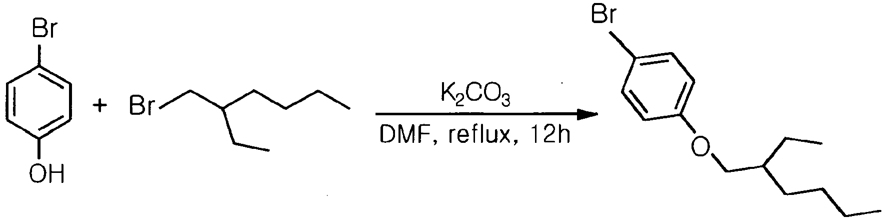

4-bromophenol (15 g, 68.18 mmol), K₂CO₃ (24.5 g, 177.26 mmol) and DMF (70 mL) were put into a 250 mL round flask, and then refluxed and stirred for 2 hours to prepare a first mixture. Then, 2-ethylhexylbromide (17.12 g, 88.63 mmol) was added to the first mixture using an injector, and then refluxed and stirred for 24 hours to prepare a second mixture. When the reaction of the second mixture was finished, the reaction mixture was cooled to room temperature, acid-treated with 70 mL of an aqueous 2 M HCl solution, extracted with methylene chloride, and then washed with distilled water several times to form an organic layer. The organic layer was dried by magnesium sulfate (MgSO₄), a solvent was removed from the dried organic layer under reduced pressure, and a reaction product was separated by column chromatography (silica, CH₂Cl₂:hexane = 3:2). The yield of the reaction product was 93%.
¹H NMR(CDCl₃. ppm): δ7.55-7.52(d, 2H, Ar-H), 6.69-6.66(d, 2H, Ar-H), 3.80-3.79(d, 2H, CH₂-O), 1.73-1.67(m, 1H,(CH₂)₃-H), 1.53-1.28(m, 8H, -CH₂), 0,97-0.88(t, 6H, -CH₃).

### 4-2: Synthesis of 2-(4-(2-ethylhexyloxy)phenyl)-4,4,5,5-tetramethyl-1,3,2-dioxaborolane

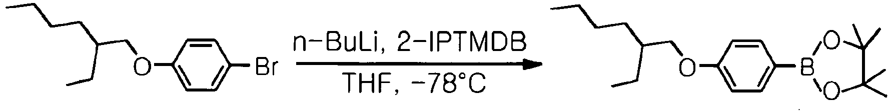

The obtained 1-bromo-4-(2-ethylhexyloxy)benzene (5.52 g, 19.4 mmol) was mixed with THF (150 mL) to prepare a first mixture, and then *n*-BuLi (1.61 g, 25.2 mmol, 2.5 min, *n*-hexane) was dripped into the first mixture at -78°C and stirred for 1 hour to prepare a second mixture. Then, 2-isopropoxy-4,4,5,5-tetramethyl-1,3,2-dioxaborolane (4.32 g, 23.2 mmol) was dripped into the second mixture, stirred for 1 hour and then further stirred at room temperature for 12 hours to prepare a third mixture. After the reaction of the third mixture, a solvent was removed, and the reaction mixture was extracted with water and ethyl acetate and dried by magnesium sulfate (MgSO₄), and then a reaction product was separated by column chromatography (*n*-hexane/DCM = 3/1). The yield of the reaction product was 92% (5.9 g).
¹HNMR(300 MHz,CDCl₃)δ7.55-7.52(d, 2H, Ar-H), 6.69-6.66(d, 2H, Ar-H), 3.80-3.79(d, 2H, CH₂-O), 1.73-1.67(m,1H,(CH₂)₃-H),1.53-1.28(m,8H,-CH₂),1.28-1.32(s,12H,-CH₃)1.53-1.28(m,8H,-CH₂),0.97-0.88(t,6H,-CH₃)

### 4-3: Synthesis of 3,6-bromo-9H-carbazole

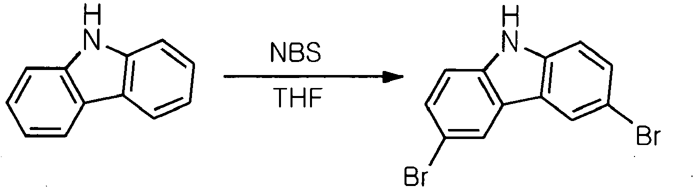

Carbazole (5.0 g, 29.9 mmol) was dissolved in THF (50 mL), and then *N-*bromosuccinimide (11.18 g, 62.80 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 83% (8.1 g).
¹HNMR(300 MHz,CDCl₃) 8.13-(s, -Ar, 2H), 7.53(d, -Ar, 2H), 7.32-7.3(d, -Ar, 2H)

### 4-4: Synthesis of 3,6-bis(4-(2-ethylhexyloxy)phenyl)-9H-carbazole

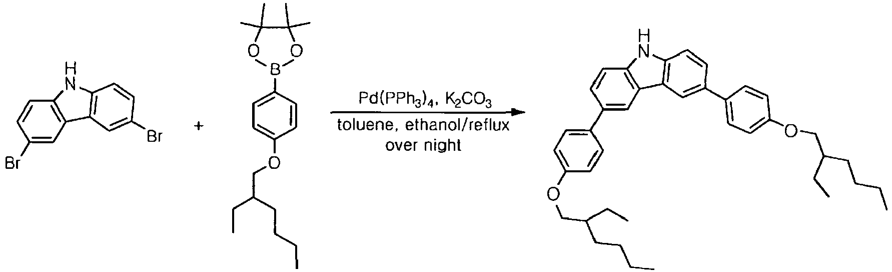

3,6-bromo-9H-carbazole (3.0 g, 9.2 mmol), 2-(4-(2-ethylhexyloxy)phenyl)-4,4,5,5-tetramethyl-1,3,2-dioxaborolane (7 g, 22 mmol), sodium carbonate (7.48 g, 42.24 mmol) and tetrakis(triphenylphosphine) palladium (Pd(PPh₃)₄) (0.68 g,0.35 mmol) were dissolved in 100 mL of toluene/tetrahydrofuran/H₂O/ethanol (3:1:1:1(v/v/v/v)) under a nitrogen atmosphere, and then refluxed and stirred at 80°C for 12 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 3/1). The yield of the reaction product was 60% (4.9 g).
¹HNMR(300 MHz,CDCl₃)δ8.27(s, Ar-H, 2H), 7.65-7.61 (d, Ar-H, 4H), 7.49-7.46(d, Ar-H, 2H), 7.03-7.00(d, Ar-H, 2H), 3.80-3.79(d, 2H, CH₂-O),1.73-1.67(m,1H,(CH₂)₃-H),1.53-1.28(m,8H,-CH₂),0.97-0.88(t,6H,-CH₃)

### 4-5: Synthesis of 4'-bromobiphenyl-4-carbonitrile

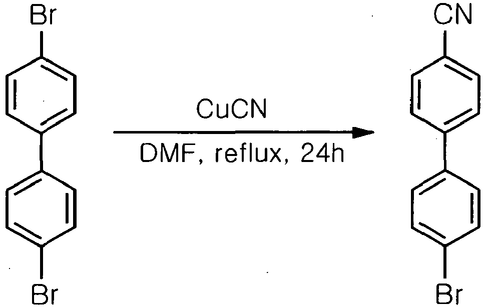

4,4'-dibromobiphenyl (10.0 g, 32.05 mmol) and copper cyanide (CuCN) (2.87 g, 32.05 mmol) were dissolved in THF (50 mL) to prepare a mixed solution. Then, the mixed solution was reacted for 24 hours in the state of reflux. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/chloroform = 1/1). The yield of the reaction product was 20% (1.7 g).
¹HNMR(300 MHz,CDCl₃) δ7.74-7.71(d, -Ar, 2H), 7.66-7.59(m, -Ar, 4H), 7.46-7.43(d, -Ar, 2H)

### 4-6: Synthesis of 4'-(3,6-bis(4-(2-ethylhexyloxy)phenyl)-9H-carbazole-9-yl)biphenyl-4-carbonitrile

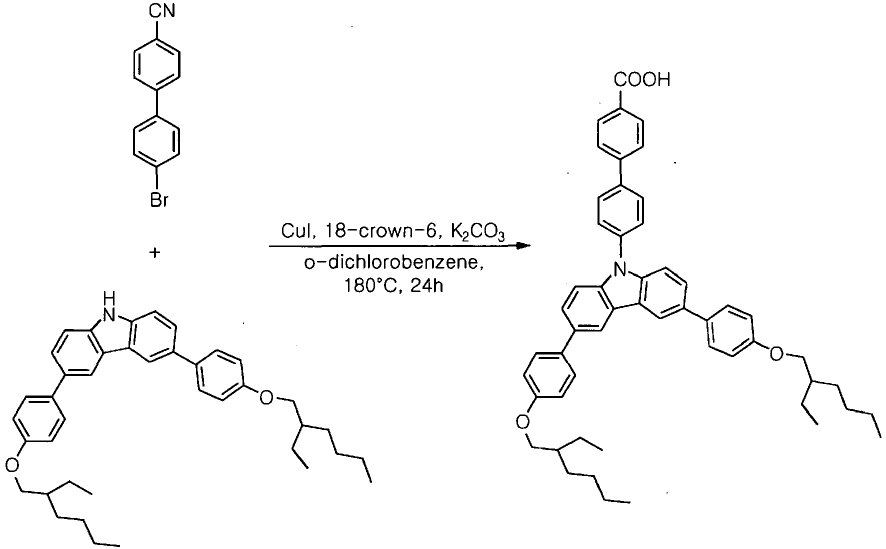

4'-bromobiphenyl-4-carbonitrile (1.9 g, 7.3 mmol) and 3,6-bis(4-(2-ethylhexyloxy)phenyl)-9H-carbazole (4.0 g, 7.42 mmol) were mixed with CuI (1.87 g, 9.83 mmol), 18-crown-6 (0.65 g, 2.46 mmol), K₂CO₃ (13.58 g, 98.25 mmol) and O-dichlorobenzene (30 ml) and then stirred at 180°C for 24 hours. After the reaction of the mixture, a solvent was removed, and the reaction mixture was extracted with water and ethyl acetate and dried by magnesium sulfate (MgSO₄), and then a reaction product was separated by column chromatography (*n*-hexane/EtOAc = 2/1). The yield of the reaction product was 64% (2.5 g).
¹HNMR(300 MHz,CDCl₃) δ8.27(s, Ar-H, 2H), 7.74-7.71(d, -Ar, 2H), 7.66-7.59(m, -Ar, 4H), 7.46-7.43(d, -Ar, 2H), 7.65-7.61(d, Ar-H, 4H), 7.49-7.46(d, Ar-H, 2H), 7.03-7.00(d, Ar-H, 2H), 3.80-3.79(d, 2H, CH₂-O), 1.73-1.67(m,1H,(CH₂)₃-H),1.53-1.28(m,8H,-CH₂),0.97-0.88(t,6H,-CH₃)

### 4-7: Synthesis of 4'-(3,6-bis(4-(2-ethylhexyloxy)phenyl)-9H-carbazole-9-yl)biphenyl-4-carboxylic acid

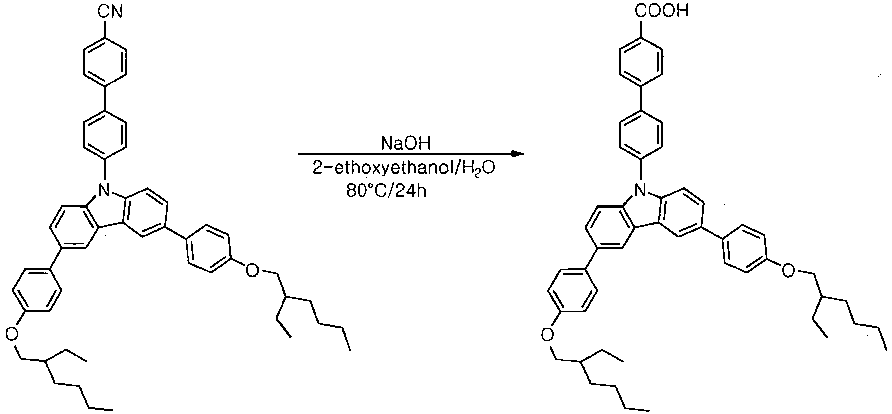

4'-(3,6-bis(4-(2-ethylhexyloxy)phenyl)-9H-carbazole-9-yl)biphenyl-4-carbonitrile (2.5 g, 3.23 mmol) and sodium hydroxide (0.51 g, 12.85 mmol) were dissolved in 20 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 2/1). The yield of the reaction product was 84% (2.0 g).
¹HNMR(300 MHz,CDCl₃) δ8.27(s, Ar-H, 2H), 7.84-7.81(d, -Ar, 2H), 7.66-7.59(m, -Ar, 4H), 7.46-7.43(d, -Ar, 2H), 7.65-7.61(d, Ar-H, 4H), 7.49-7.46(d, Ar-H, 2H), 7.03-7.00(d, Ar-H, 2H), 3.80-3.79(d, 2H, CH₂-O), 1.73-1.67(m,1H,(CH₂)₃-H),1.53-1.28(m,8H,-CH₂),0.97-0.88(t,6H,-CH₃)

### Comparative Example 5: Synthesis of 4,4'-(3,6-bis(4-(2-ethylhexyloxy)phenyl)-9H-carbazole-9-yl)-2,2',6,6'-tetramethylbiphenyl-4-carboxylic acid

### 5-1: Synthesis of 1-bromo-4-(2-ethylhexyloxy)benzene

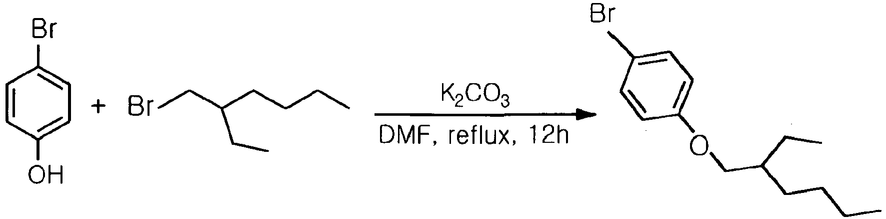

4-bromophenol (15 g, 68.18 mmol), K₂CO₃ (24.5 g, 177.26 mmol) and DMF (70 mL) were put into a 250 mL round flask, and then refluxed and stirred for 2 hours to prepare a first mixture. Then, 2-ethylhexylbromide (17.12 g, 88.63 mmol) was added to the first mixture using an injector, and then refluxed and stirred for 24 hours to prepare a second mixture. When the reaction of the second mixture was finished, the reaction mixture was cooled to room temperature, acid-treated with 70 mL of an aqueous 2 M HCl solution, extracted with methylene chloride, and then washed with distilled water several times to form an organic layer. The organic layer was dried by magnesium sulfate (MgSO₄), a solvent was removed from the dried organic layer under reduced pressure, and a reaction product was separated by column chromatography (silica, CH₂Cl₂:hexane = 3:2). The yield of the reaction product was 93%.
¹H NMR(CDCl₃. ppm): δ7.55-7.52(d, 2H, Ar-H), 6.69-6.66(d, 2H, Ar-H), 3.80-3.79(d, 2H, CH₂-O), 1.73-1.67(m, 1H,(CH₂)₃-H), 1.53-1.28(m, 8H, -CH₂), 0.97-0.88(t, 6H, -CH₃).

### 5-2: Synthesis of 2-(4-(2-ethylhexyloxy)phenyl)-4,4,5,5-tetramethyl-1,3,2-dioxaborolane

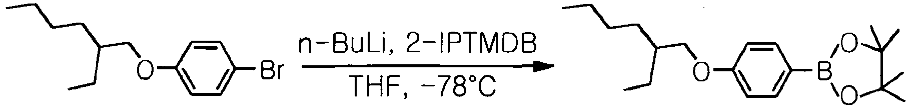

The obtained 1-bromo-4-(2-ethylhexyloxy)benzene (5.52 g, 19.4 mmol) was mixed with THF (150 mL) to prepare a first mixture, and then n-BuLi (1.61 g, 25.2 mmol, 2.5 min, *n*-hexane) was dripped into the first mixture at -78°C and stirred for 1 hour to prepare a second mixture. Then, 2-isopropoxy-4,4,5,5-tetramethyl-1,3,2-dioxaborolane (4.32 g, 23.2 mmol) was dripped into the second mixture, stirred for 1 hour and then further stirred at room temperature for 12 hours to prepare a third mixture. After the reaction of the third mixture, a solvent was removed, and the reaction mixture was extracted with water and ethyl acetate and dried by magnesium sulfate (MgSO₄), and then a reaction product was separated by column chromatography (*n*-hexane/DCM = 3/1). The yield of the reaction product was 92% (5.9 g).
¹HNMR(300 MHz,CDCl₃)δ7.55-7.52(d, 2H, Ar-H), 6.69-6.66(d, 2H, Ar-H), 3.80-3.79(d, 2H, CH₂-O), 1.73-1.67(m,1H,(CH₂)₃-H),1.53-1.28(m,8H,-CH₂),1.28-1.32(s,12H,-CH₃)1.53-1.28(m,8H,-CH₂),0.97-0.88(t,6H,-CH₃)

### 5-3: Synthesis of 3,6-bromo-9H-carbazole

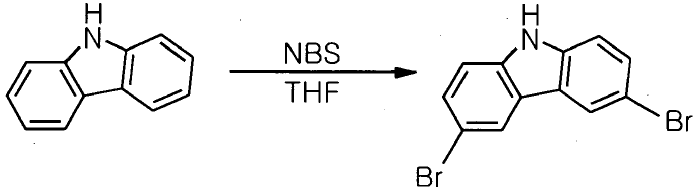

Carbazole (5.0 g, 29.9 mmol) was dissolved in THF (50 mL), and then *N-*bromosuccinimide (11.18 g, 62.80 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 83% (8.1 g).
¹HNMR(300 MHz,CDCl₃) 8.13-(s, -Ar, 2H), 7.53(d, -Ar, 2H), 7.32-7.3(d, -Ar, 2H)

### 5-4: Synthesis of 3,6-bis(4-(2-ethylhexyloxy)phenyl)-9H-carbazole

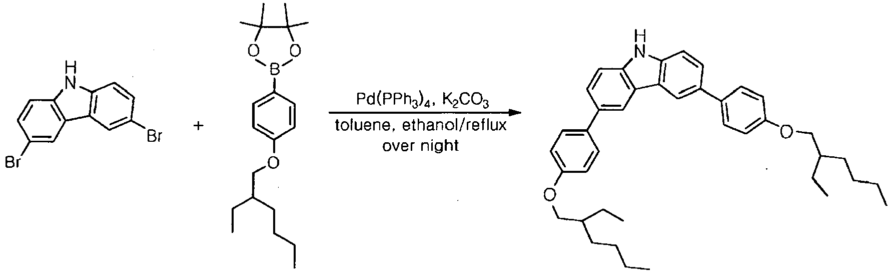

3,6-bromo-9H-carbazole (3.0 g, 9.2 mmol), 2-(4-(2-ethylhexyloxy)phenyl)-4,4,5,5-tetramethyl-1,3,2-dioxaborolane (7 g, 22 mmol), sodium carbonate (7.48 g, 42.24 mmol) and tetrakis(triphenylphosphine) palladium (Pd(PPh₃)₄) (0.68 g, 0.35 mmol) were dissolved in 100 mL of toluene/tetrahydrofuran/H₂O/ethanol (3:1:1:1(v/v/v/v)) under a nitrogen atmosphere, and then refluxed and stirred at 80 °C for 12 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 3/1). The yield of the reaction product was 60% (4.9 g).
¹HNMR(300 MHz,CDCl₃)δ8.27(s, Ar-H, 2H), 7.65-7.61(d, Ar-H, 4H), 7.49-7.46(d, Ar-H, 2H), 7.03-7.00(d, Ar-H, 2H), 3.80-3.79(d, 2H, CH₂-O),1.73-1.67(m,1H,(CH₂)₃-H),1.53-1.28(m,8H,-CH₂),0.97-0.88(t,6H,-CH₃)

### 5-5: Synthesis of 1,2-bis(3,5-dimethylphenyl)hydrazine

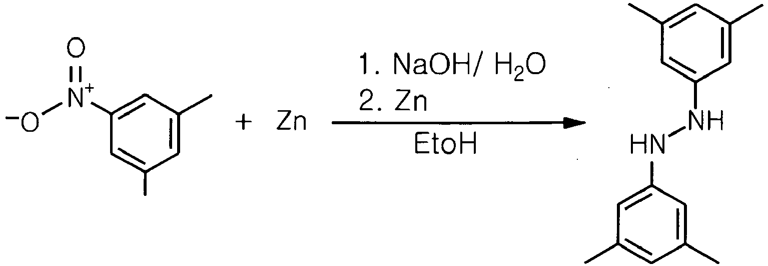

3,5-dimethylnitrobenzene (10 g, 66.16 mmol) and zinc powder (25 g, 383.70 mmol) were mixed with ethanol (200 mL), and then heated while injecting argon to prepare a first mixed solution. NaOH (5.7 equivalents, 15.0 g, 375 mmol) was dissolved in water (50 mL) to prepare a second mixed solution. The second mixed solution was dripped into the first mixed solution little by little, and simultaneously 10 g of zinc powder was gradually added to the first mixed solution for 4 hours to prepare a third mixed solution. Thereafter, acetic acid (150 mL, 30%) and sodium bisulfite (1.0 g, 9.6 mmol, 0.15 equivalents) were added to the third mixed solution and then stirred to prepare a fourth mixed solution. Then, the fourth mixed solution was filtered using a filter paper while pouring hot ethanol (60 mL).

The filtered mixed solution was cooled to obtain crystals. The obtained crystals were filtered by a filter paper, dissolved in a heptane solution (80 mL) and then cooled again to be recrystallized. The yield thereof was 20.5% (2.3 g).
¹HNMR(300 MHz,CDCl₃)δ6.46(s, Ar-H, 6H), 5.42(s, N-H, 2H), 2.20(s, CH₃, 12H)

### 5-6: Synthesis of 2,2',6,6'-tetramethylbiphenyl-4,4'-diamine

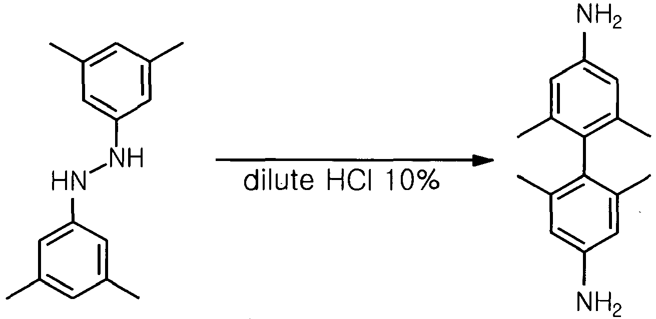

1,2-bis(3,5-dimethylphenyl)hydrazine (2.0 g, 8.32 mmol) was mixed with an aqueous hydrochloric acid solution (10%, 200 mL), and then a reaction was conducted for 2 hours to obtain a reaction product. Then, sodium hydride was added to the reaction product to adjust the pH of the reaction product to 10. The reaction product was extracted with tert-butyl-methyl-ether and water, dried by magnesium sulfate (MgSO₄), and then washed with hexane. The yield of the reaction product was 64% (1.6 g).
¹HNMR(300 MHz,CDCl₃)δ6.47(s, Ar-H, 4H), 3.58(br, N-H, 4H), 1.79(s, CH₃, 12H)

### 5-7: Synthesis of 4,4'-diiodo-2,2',6,6'-tetramethylbiphenyl

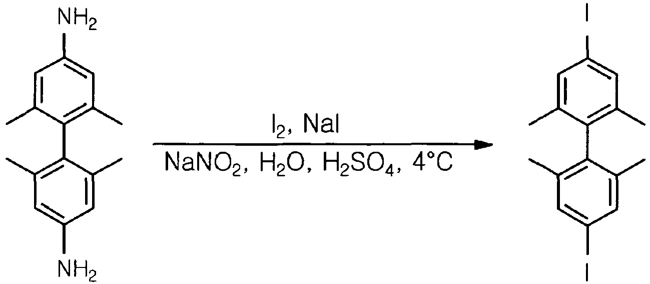

2,2',6,6'-tetramethylbiphenyl-4,4'-diamine (1.6 g, 6.66 mmol) and NaNO₂ (0.6 g, 8.70 mmol) were dissolved in water (20 mL) to prepare an aqueous solution, and then the prepared aqueous solution was added to a mixed solution of water (4 °C, 15 mL) and sulfuric acid (8 ml, 147 mmol) and stirred for 30 minutes to prepare a first aqueous mixed solution. The first aqueous mixed solution was added to an aqueous solution obtained by dissolving I₂ (2.90 g, 10.64 mmol) and NaI (2.70 g 18.00 mmol) in water (5 mL) and then performing a reaction for 30 minutes, stirred for 20 minutes, and then water (20 mL) and MC (50 mL) were added thereto and the stirred for 24 hours to perform a reaction to obtain a reaction product. After the reaction, sodium thiosulfate was added to the reaction product, stirred for 10 minutes, filtered by a filter paper, and then extracted with MC. The extracted reaction product was dried by magnesium sulfate (MgSO₄), and then separated by column chromatography (n-hexane). The yield of the reaction product was 6% (2.0 g).
¹HNMR(300 MHz,CDCl₃) δ7.48(s, -Ar, 4H), 1.94(s, CH₃, 12H)

### 5-8: Synthesis of 4'-iodo-2,2',6,6'-tetramethylbiphenyl-4-carbonitrile

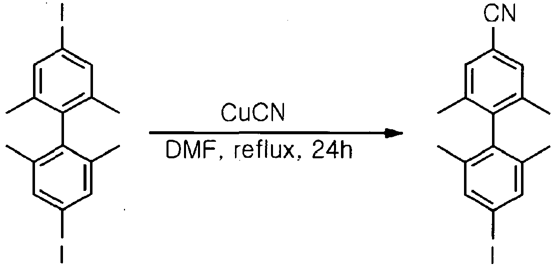

4,4'-diiodo-2,2',6,6'-tetramethylbiphenyl (2.0 g, 4.33 mmol) and copper cyanide (CuCN) (0.39 g, 4.33 mmol) were dissolved in THF (50 mL) to prepare a mixed solution. Then, the mixed solution was reacted for 24 hours in the state of reflux. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/chloroform = 1/1). The yield of the reaction product was 40% (0.7 g).
¹HNMR(300 MHz,CDCl₃) δ7.51(s, -Ar, 2H), 7.43(s, -Ar, 2H), 7.46-7.43(d, - Ar, 2H), 1.94(s, CH₃, 6H), 1.84(s, CH₃, 6H)

### 5-9: Synthesis of 4'-(3,6-bis(4-(2-ethylhexyloxy)phenyl)-9H-carbazole-9-yl)-2,2',6,6'-tetramethylbiphenyl-4-carbonitrile

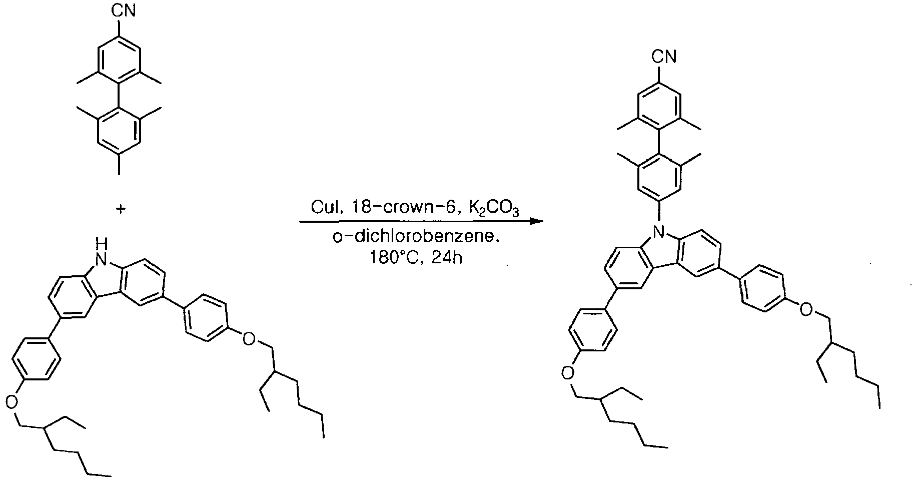

4'-iodo-2,2',6,6'-tetramethylbiphenyl-4-carbonitrile (0.7 g, 1.94 mmol) and 3,6-bis(4-(2-ethylhexyloxy)phenyl)-9H-carbazole (1.45 g, 2.54 mmol) were mixed with CuI (1.87 g, 9.83 mmol), 18-crown-6 (0.65 g, 2.46 mmol), K₂CO₃(13.58 g, 98.25 mmol) and O-dichlorobenzene (30 ml) and then stirred at 180°C for 24 hours. After the reaction of the mixture, a solvent was removed, and the reaction mixture was extracted with water and ethyl acetate and dried by magnesium sulfate (MgSO₄), and then a reaction product was separated by column chromatography (*n*-hexane/EtOAc = 2/1). The yield of the reaction product was 54% (0.8 g).
¹HNMR(300 MHz,CDCl₃) δ7.51(s, -Ar, 2H), 7.43(s, -Ar, 2H), 7.46-7.43(d,-Ar, 2H), 1.94(s, CH₃, 6H), 1.84(s, CH₃, 6H),δ8.27(s, Ar-H, 2H), 7.65-7.61(d, Ar-H, 4H), 7.49-7.46(d, Ar-H, 2H), 7.03-7.00(d, Ar-H, 2H), 3.80-3.79(d, 2H, CH₂-O),1.73-1.67(m,1H,(CH₂)₃-H),1.53-1.28(m,8H,-CH₂),0.97-0.88(t,6H,-CH₃)

### 5-10: Synthesis of 4,4'-(3,6-bis(4-(2-ethylhexyloxy)phenyl)-9H-carbazole-9-yl)-2,2',6,6'-tetramethylbiphenyl-4-carboxylic acid

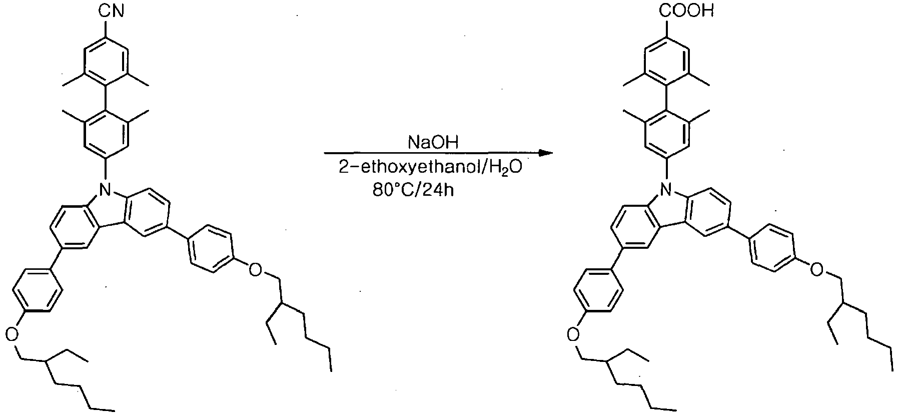

4'-(3,6-bis(4-(2-ethylhexyloxy)phenyl)-9H-carbazole-9-yl)-2,2',6,6'-tetramethylbiphenyl-4-carbonitrile (0.8 g, 0.98 mmol) and sodium hydroxide (0.51 g, 12.85 mmol) were dissolved in 20 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 2/1). The yield of the reaction product was 84% (0.7 g).
¹HNMR(300 MHz,CDCl₃) δ8.27(s, Ar-H, 2H), 7.74-7.81(d, -Ar, 2H), 7.66-7.59(m, -Ar, 4H), 7.46-7.43(d, -Ar, 2H), 7.65-7.61(d, Ar-H, 4H), 7.49-7.46(d, Ar-H, 2H), 7.03-7.00(d, Ar-H, 2H), 3.80-3.79(d, 2H, CH₂-O),1.73-1.67(m,1H,(CH₂)₃-H),1.53-1.28(m,8H,-CH₂),0.97-0.88(t,6H,-CH₃)

### Comparative Example 6: Synthesis of 4-(3,6-bis(4-2,5,8,11-tetraoxadodecylphenyl)-9H-carbazole-9-yl)benzoic acid

### 6-1: Synthesis of 1-(4-bromophenyl)-2,5,8,11-tetraoxadodecane

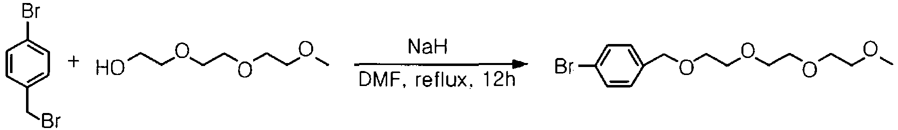

2-(2-(2-methoxyethoxy)ethoxy)ethanol (10 g, 60.9 mmol) and NaH (3.17 g, 79.17 mmol) were mixed with DMF (50 mL) and then stirred at room temperature for 1 hour to prepare a first mixture. Then, 1-bromo-4-(bromoethyl)benzene (14.46 g, 57.86 mmol) was dripped into the first mixture and then stirred at room temperature for 23 hours to prepare a second mixture. After the reaction of the second mixture, the reaction mixture was extracted with water and ethyl acetate and dried by magnesium sulfate (MgSO₄), and then a reaction product was separated by column chromatography (*n*-hexane/ DCM = 2/1). The yield of the reaction product was 64% (13 g).
¹HNMR(300 MHz,CDCl₃)δ7.46-7.35(d, 2H, Ar-H), 7.22-7.19(d, 2H, Ar-H), 4.50(s, 2H, Ar-CH₂-O),3.72-3.67(m,12H,O-CH₂),3.35-3.34(s,3H,O-CH₃)

### 6-2: Synthesis of 2-(4-2,5,8,11-tetraoxadodecylphenyl)-4,4,5,5-tetramethyl-1,3,2-dioxaborolane

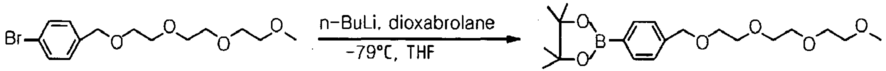

The obtained 1-(4-bromophenyl)-2,5,8,11-tetraoxadodecane (13 g, 39.01 mmol) was mixed with THF (150 mL) to prepare a first mixture, and then *n*-BuLi (3.00 g, 46.82 mmol, 1.6 Min, *n*-hexane) was dripped into the first mixture at -79°C and stirred for 1 hour to prepare a second mixture. Then, 2-isopropoxy-4,4,5,5-tetramethyl-1,3,2-dioxaborolane (9.44 g, 50.72 mmol) was dripped into the second mixture, stirred for 1 hour and then further stirred at room temperature for 12 hours to prepare a third mixture. After the reaction of the third mixture, a solvent was removed, and the reaction mixture was extracted with water and ethyl acetate and dried by magnesium sulfate (MgSO₄), and then a reaction product was separated by column chromatography (*n*-hexane/DCM = 3/1). The yield of the reaction product was 92% (5.9 g).
¹HNMR(300 MHz,CDCl₃)δ7.78-7.75(d, 2H, Ar-H), 7.22-7.19(d, 2H, Ar-H), 4.50(s, 2H, Ar-CH₂-O),3.72-3.67(m, 12H,O-CH₂),3.35-3.34(s,3H,O-CH₃)

### 6-3: Synthesis of 4-(9H-carbazole-9-yl)benzonitrile

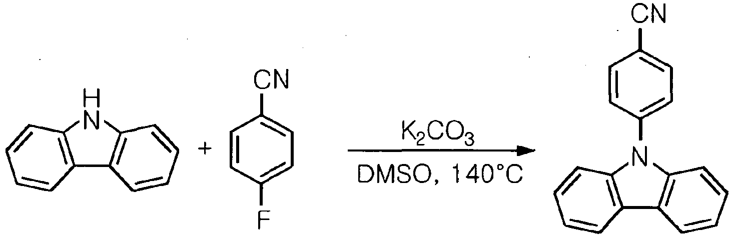

9H-carbazole (3 g, 17.94 mmol) was mixed with K₂CO3(8.22 g, 26.91 mmol) and DMSO (30 mL) and then stirred at room temperature for 1 hour to prepare a first mixture. Then, 4-fluorobenzonitrile (2.61 g, 21.5 mmol) was dripped into the first mixture and then stirred at 140°C for 12 hours to prepare a second mixture. After the reaction of the second mixture, a solvent was removed, the reaction mixture was extracted with water and ethyl acetate and dried by magnesium sulfate (MgSO₄), and then a reaction product was separated by column chromatography (*n*-hexane/EtOAc = 2/1). The yield of the reaction product was 94% (4.5 g).
¹HNMR(300 MHz,CDCl₃) 8.13(d, Ar-H, 2H), 7.44(d, Ar-H, 2H), 7.38(m, Ar-H, 2H), 7.32(d, Ar-H, 2H), 7.27(m, Ar-H, 2H), 7.10(d, Ar-H, 2H)

### 6-4: Synthesis of 4-(3,6-dibromo-9H-carbazole-9-yl)benzonitrile

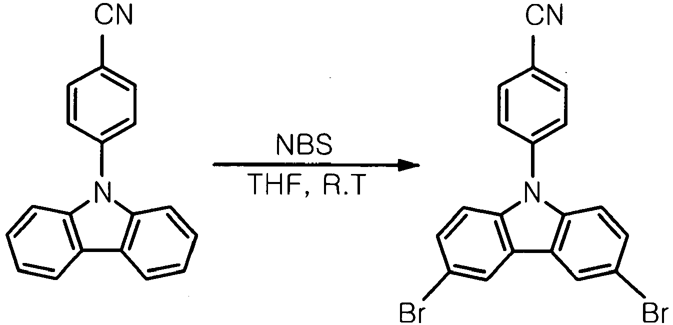

4-(9H-carbazole-9-yl)benzonitrile (4.5 g, 16.8 mmol) was dissolved in THF (50 mL), and then *N*-bromosuccinimide (5.9 g, 33.9 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 90% (6.5g).
¹HNMR(300 MHz,CDCl₃)δ7.01-7.03(d,-Ar,4H),7.48-7.51(d,-Ar,2H), 8.27(s,-Ar,2H)

### 6-5: Synthesis of 4-(3,6-bis(4-2,5,8,11-tetraoxadodecylphenyl)-9H-carbazole-9-yl)benzonitrile

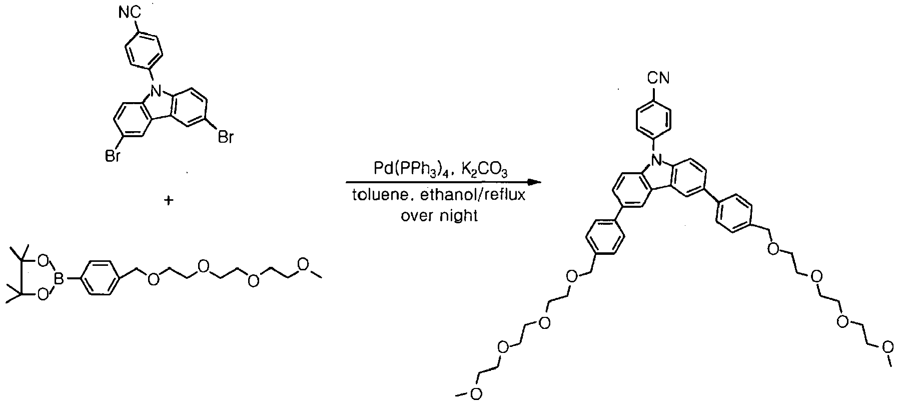

4-(3,6-dibromo-9H-carbazole-9-yl)benzonitrile (3.0 g, 7.04 mmol), 2-(4-2,5,8,11-tetraoxadodecylphenyl)-4,4,5,5-tetramethyl-1,3,2-dioxaborolane (5.89 g, 15.49 mmol), sodium carbonate (4.86 g, 35.20 mmol) and tetrakis(triphenylphosphine) palladium (Pd(PPh₃)₄) (0.41 g, 0.35 mmol) were dissolved in 100 mL of toluene/tetrahydrofuran/H₂O/ethanol (3:1:1:1(v/v/v/v)) under a nitrogen atmosphere, and then refluxed and stirred at 80°C for 12 hours to prepare a mixed solution. After the reaction of the mixed solution, the reacted mixed solution was extracted with water and ethyl acetate and dried by magnesium sulfate (MgSO₄), and then a reaction product was separated by column chromatography (*n-*hexane/EtOAc = 3/1). The yield of the reaction product was 53% (2.9 g).
¹HNMR(300 MHz,CDCl₃)δ7.01-7.03(d,-Ar,4H),7.48-7.51(d,-Ar,2H), 8.27(s,-Ar,2H), δ7.91-7.88(d, 2H, Ar-H), 7.22-7.19(d, 2H, Ar-H), 4.50(s, 2H, Ar-CH₂-O),3.72-3.67(m,12H,O-CH2),3.35-3.34(s,3H,O-CH₃)

### 6-6: Synthesis of 4-(3,6-bis(4-2,5,8,11-tetraoxadodecylphenyl)-9H-carbazole-9-yl)benzoic acid

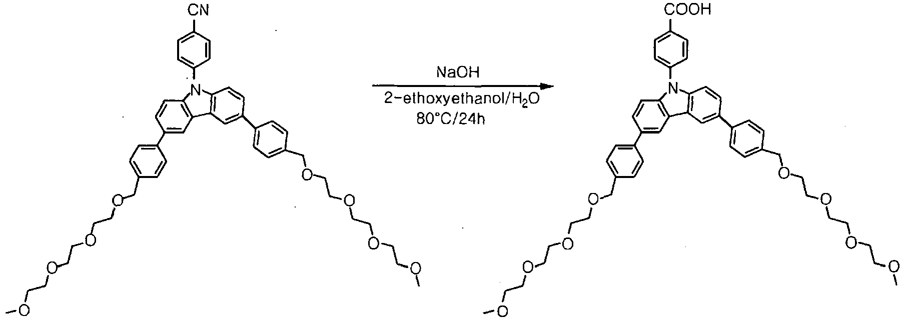

4-(3,6-bis(4-2,5,8,11-tetraoxadedocylphenyl)-9H-carbazole-9-yl)benzonitrile (2.9 g, 3.75 mmol) and sodium hydroxide (0.51 g, 12.85 mmol) were dissolved in 20 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (*n-*hexane/EtOAc = 2/1). The yield of the reaction product was 94%(4.5 g).
¹HNMR(300 MHz,CDCl₃)δ7.01-7.03(d,-Ar,4H),7.48-7.51(d,-Ar,2H), 8.27(s,-Ar,2H), δ7.91-7.88(d, 2H, Ar-H), 7.22-7.19(d, 2H, Ar-H), 4.50(s, 2H, Ar-CH₂-O),3.72-3.67(m,12H,O-CH₂),3.35-3.34(s,3H,O-CH₃)

### Comparative Example 7: Synthesis of 3,5-bis(3,6-bis(4-methoxyphenyl)-9H-carbazole-9-yl)benzoic acid

### 7-1: Synthesis of 3,5-di(9H-carbazole-9-yl)benzonitrile

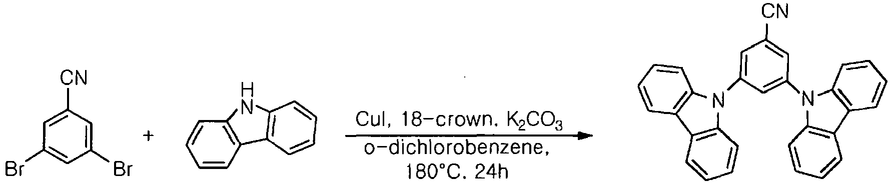

9H-carbazole (6.4 g, 40.00 mmol) was mixed with K₂CO₃ (10.6 g, 76.64 mmol), CuI (0.91 g, 4.79 mmol), 3,5-dibromobenzonitrile (5 g, 19.16 mmol), 18-crown-6 (1.01 g, 3.83 mmol), and O-dichlorobenzene (40 mL) and then stirred at 180°C for 24 hours. After the reaction of the mixture, salts and copper (Cu) were filtered out by a filter paper, a solvent was removed, and the reaction mixture was extracted with water and ethyl acetate and dried by magnesium sulfate (MgSO₄), and then a reaction product was separated by column chromatography (*n*-hexane/EtOAc = 2/1). The yield of the reaction product was 60% (4.9 g).
¹HNMR(300 MHz,CDCl₃) 8.13(d, -Ar, 4H), 7.50(s, -Ar, 3H), 7.08-7.20(d, -Ar, 8H) 7.27(m, -Ar, 4H)

### 7-2: Synthesis of 3,5-bis(3,6-dibromo-9H-carbazole-9-yl)benzonitrile

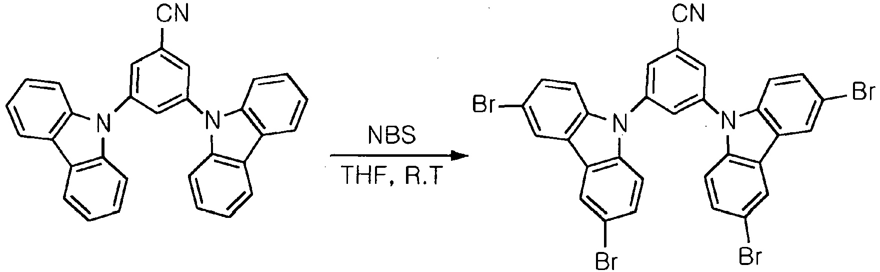

3,5-di(9H-carbazole-9-yl)benzonitrile (4.9 g, 16.8 mmol) was dissolved in THF (50 mL), and then *N*-bromosuccinimide (8.25 g, 46.34 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 80% (6.8 g).
¹HNMR(300 MHz,CDCl₃) 8.21(d, -Ar, 4H), 7.50(s, -Ar, 3H), 7.25-7.29(d, Ar-H, 8H)

### 7-3: Synthesis of 4-methoxyphenylboronic acid

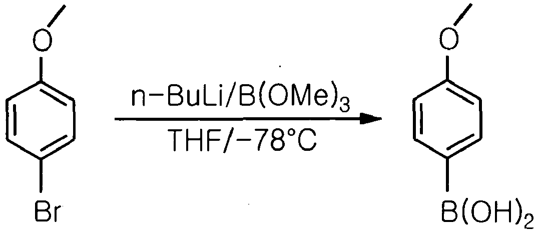

1-bromo-4-methoxybenzene (15.0 g, 80.2 mmol) was dissolved in tetrahydrofuran (150 mL) to prepare a first solution, and then butyl lithium (5.65 g, 88.2 mmol) was slowly dripped into the first solution at -78°C and then stirred for 1 hour to prepare a second solution. Subsequently, trimethyl borate (16.7 g, 160.4 mmol) was slowly dripped into the second solution, stirred at -78°C for 1 hour and then stirred at room temperature during the night to prepare a third solution. After the reaction of the third solution, the pH of the reaction solution was adjusted to 2 by 6M-HCl, the reaction solution was extracted with water and ethyl acetate and then separated into an organic solvent layer and an aqueous solution layer, and then the organic solvent layer was dried by anhydrous magnesium sulfate and filtered. Then, an organic solvent was volatilized, a residue was dissolved in a small amount of ether, and then n-hexane was dripped into the dissolved residue at 0°C to precipitate crystals. The crystals were filtered and dried. The yield thereof was 66% (8.0 g).
¹H NMR(300MHz, CDCl₃) δ 3.81(s, -OCH₃, 3H), 6.89-6.91(d, -Ar, 2H), 7.72-7.74(d, -Ar, 2H)

### 7-4: Synthesis of 3,5-bis(3,6-bis(4-methoxyphenyl)-9H-carbazole-9-yl)benzonitrile

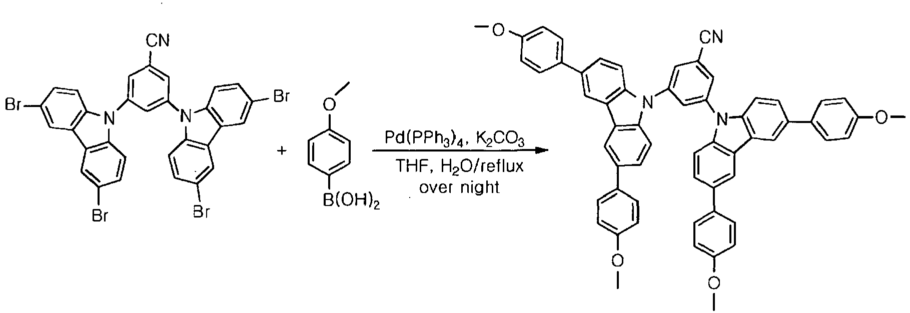

3,5-bis(3,6-dibromo-9H-carbazole-9-yl)benzonitrile (6.8 g, 15.69 mmol), 4-methoxyphenylboronic acid (10.75 g, 64.31 mmol), potassium carbonate (17.34 g, 125.49 mmol) and tetrakis(triphenylphosphine)palladium (3.63 g, 3.14 mmol) were dissolved in 100 mL of THF/H₂O (10:3) to prepare a mixed solution. After the reaction of the mixed solution at 80°C, the reacted mixed solution was extracted with water and ethyl acetate and dried by anhydrous magnesium sulfate and filtered, and then an organic solvent was volatilized, and a reaction product was separated by column chromatography (*n*-hexane/EtOAc = 2/1). The yield of the reaction product was 54%(7.3 g).
¹HNMR(300 MHz,CDCl₃) 8.21(d, -Ar, 4H), 7.50(s, -Ar, 3H), 7.25-7.29(d, -Ar, 8H), 3.8(s, CH₃-O, 12H), 6.89-6.91(d, -Ar, 8H), 7.72-7.74(d, -Ar, 8H)

### 7-5: Synthesis of 3,5-bis(3,6-bis(4-methoxyphenyl)-9H-carbazole-9-yl)benzoic acid

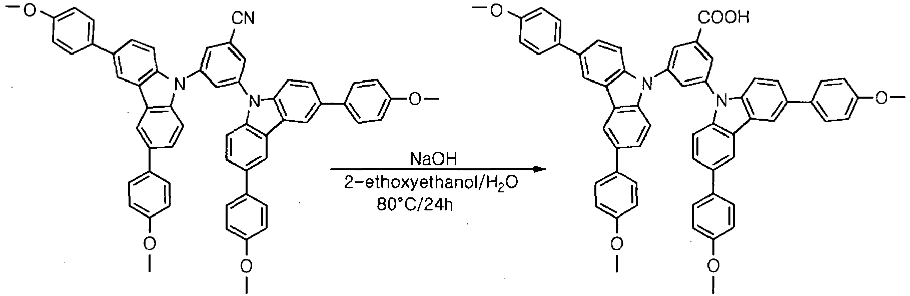

3,5-bis(3,6-bis(4-methoxyphenyl)-9H-carbazole-9-yl)benzonitrile (7.3 g, 7.93 mmol) and sodium hydroxide (0.95 g, 23.78 mmol) were dissolved in 40 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 3/1). The yield of the reaction product was 80% (5.5 g).
¹HNMR(300 MHz,CDCl₃) 8.21(d, -Ar, 4H), 7.50(s, -Ar, 3H), 7.25-7.29(d, Ar-H, 8H), 3.8(s, CH₃-O, 12H), 6.89-6.91(d, -Ar, 8H), 7.72-7.74(d, -Ar, 8H), 12.7(s, OH, H)

### Comparative Example 8: Synthesis of 9-[3,5-bis(phenyl)-3,6-bis(4-methoxyphenyl)]-9H-carbazole-9-yl]benzoic acid

### 8-1: Synthesis of 4-methoxyphenylboronic acid

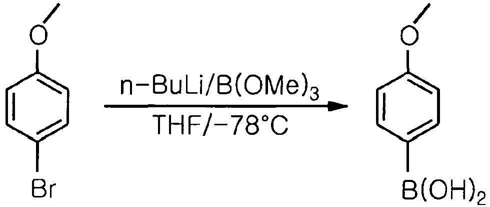

1-bromo-4-methoxybenzene (15.0 g, 80.2 mmol) was dissolved in tetrahydrofuran (150 mL) to prepare a first solution, and then butyl lithium (5.65 g, 88.2 mmol) was slowly dripped into the first solution at -78°C and then stirred for 1 hour to prepare a second solution. Subsequently, trimethyl borate (16.7 g, 160.4 mmol) was slowly dripped into the second solution, stirred at -78°C for 1 hour and then stirred at room temperature during the night to prepare a third solution. After the reaction of the third solution, the pH of the reaction solution was adjusted to 2 by 6M-HCl, the reaction solution was extracted with water and ethyl acetate and then separated into an organic solvent layer and an aqueous solution layer, and then the organic solvent layer was dried by anhydrous magnesium sulfate and filtered. Then, an organic solvent was volatilized, and a residue was dissolved in a small amount of ether, and then n-hexane was dripped into the dissolved residue at 0°C to precipitate crystals. The crystals were filtered and dried. The yield thereof was 66% (8.0 g).
¹H NMR(300MHz, CDCl₃) δ 3.81(s, -OCH₃, 3H), 6.89-6.91(d, -Ar, 2H), 7.72-7.74(d, -Ar, 2H)

### 8-2: Synthesis of 5-cyano-1,3-phenylenediboronic acid

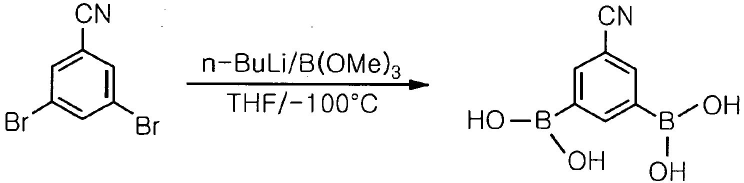

3,5-dibromobenzonitrile (15.0 g, 57.49 mmol) was dissolved in tetrahydrofuran (150 mL) to prepare a first solution, and then butyl lithium (8.10 g, 126.48 mmol) was slowly dripped into the first solution at -100°C and then stirred for 1 hour to prepare a second solution. Subsequently, trimethyl borate (23.90 g, 229.96 mmol) was slowly dripped into the second solution, stirred at -100°C for 1 hour and then stirred at room temperature during the night to prepare a third solution. After the reaction of the third solution, the pH of the reaction solution was adjusted to 2 by 6M-HCl, the reaction solution was extracted with water and ethyl acetate and then separated into an organic solvent layer and an aqueous solution layer, and then the organic solvent layer was dried by anhydrous magnesium sulfate and filtered. Then, an organic solvent was volatilized, a residue was dissolved in a small amount of ether, and then n-hexane was dripped into the dissolved residue at 0°C to precipitate crystals. The crystals were filtered and dried. The yield thereof was 74% (8.0 g).
¹H NMR(300MHz, CDCl₃) δ 7.54(s, -Ar, 3H), 2.0(s, OH, 4H)

### 8-3: Synthesis of 3,6-dibromo-9H-carbazole

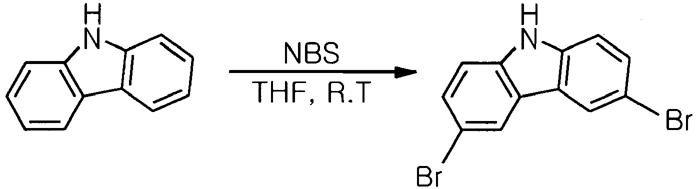

Carbazole (5.0 g, 29.9 mmol) was dissolved in THF (50 mL), and then *N-*bromosuccinimide (11.18 g, 62.80 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 83% (8.1 g).
¹HNMR(300 MHz,CDCl₃)8.21(d, -Ar, 2H), 7.25-7.29(d, -Ar, 4H), 11.70(s, N-H, H)

### 8-4: Synthesis of 3,6-bis(4-methoxyphenyl)-9H-carbazole

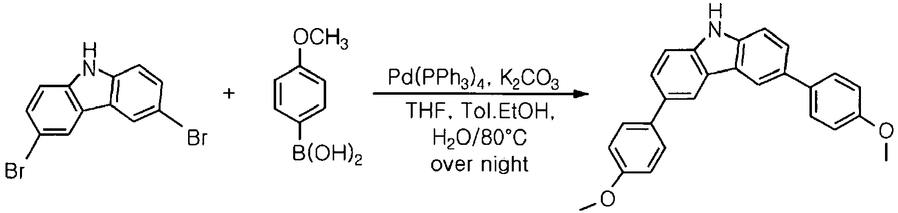

3,6-dibromo-9H-carbazole (8.1 g, 24.92 mmol), 4-methoxyphenylboronic acid (8.75 g, 52.34 mmol), potassium carbonate (10.33 g, 77.77 mmol) and tetrakis(triphenylphosphine)palladium (2.88 g, 2.49 mmol) were dissolved in 100 mL of THF/toluene/ethanol/H₂O (bpb = 1:1:1:1) to prepare a mixed solution. After the reaction of the mixed solution at 80°C, the reacted mixed solution was extracted with water and ethyl acetate and dried by anhydrous magnesium sulfate and filtered, an organic solvent was volatilized, and a reaction product was separated by column chromatography (*n*-hexane/EtOAc = 3/1). The yield of the reaction product was 54% (7.3 g).
¹HNMR(300 MHz,CDCl₃) 8.21(d, -Ar, 2H), 7.25-7.29(d, -Ar, 4H), 3.8(s, CH₃-O, 12H), 7.02(d, -Ar, 2H), 7.65(d, -Ar, 2H), 11.70(s, N-H, H)

### 8-5: Synthesis of 3,6-bis(4-methoxyphenyl)-9-phenyl-9H-carbazole

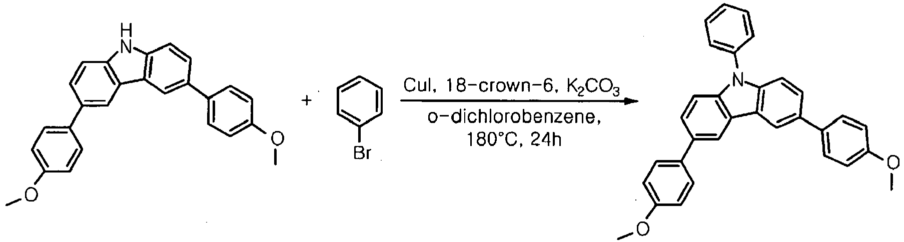

3,6-bis(4-methoxyphenyl)-9H-carbazole (7.3 g, 19.24 mmol) was mixed with K₂CO₃(5.32 g, 38.48 mmol), CuI (0.44 g, 2.31 mmol), bromobenzene (3.32 g, 21.16 mmol), 18-crown-6 (0.51 g, 1.91 mmol) and O-dichlorobenzene (50 mL) and then stirred at 180°C for 24 hours. After the reaction of the mixture, salts and copper (Cu) were filtered out by a filter paper, a solvent was removed, the reaction mixture was extracted with water and ethyl acetate and dried by magnesium sulfate (MgSO₄), and then a reaction product was separated by column chromatography (*n*-hexane/EtOAc = 3/1). The yield of the reaction product was 74% (6.5 g).
¹HNMR(300 MHz,CDCl₃) 8.21(d, -Ar, 2H), 7.25-7.29(d, -Ar, 4H), 3.8(s, CH₃-O, 12H), 7.02(d, -Ar, 2H), 7.65(d, -Ar, 2H), 7.55(m, -Ar, 2H), 7.50(m, -Ar, H), 7.30(d, -Ar, 2H)

### 8-6: Synthesis of 9-(4-bromophenyl)-3,6-bis(4-methoxyphenyl)-9H-carbazole

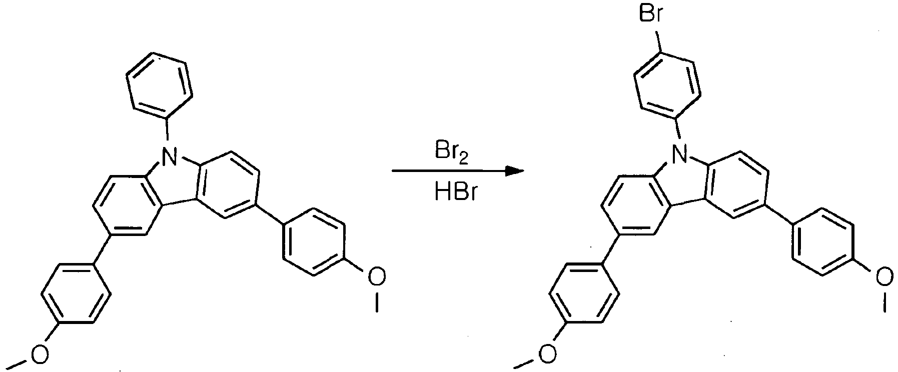

3,6-bis(4-methoxyphenyl)-9-phenyl-9H-carbazole (6.5 g, 14.27 mmol) was mixed with HBr (50 mL), and then Br₂ (3.42 g, 21.40 mmol) was dripped thereinto and then stirred for 12 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 2/1). The yield of the reaction product was 78% (6.0 g).
¹HNMR(300 MHz,CDCl₃) 8.21(d, -Ar, 2H), 7.25-7.29(d, -Ar, 4H), 3.8(s, CH₃-O, 6H), 7.02(d, -Ar, 2H), 7.65(d, -Ar, 2H), 7.4(d, -Ar, 2H), 7.2(d, -Ar, 2H)

### 8-7: Synthesis of 3,5-bis[4-(3,6-bis(4-methoxyphenyl)-9H-carbazole-9-yl)phenyl]benzonitrile

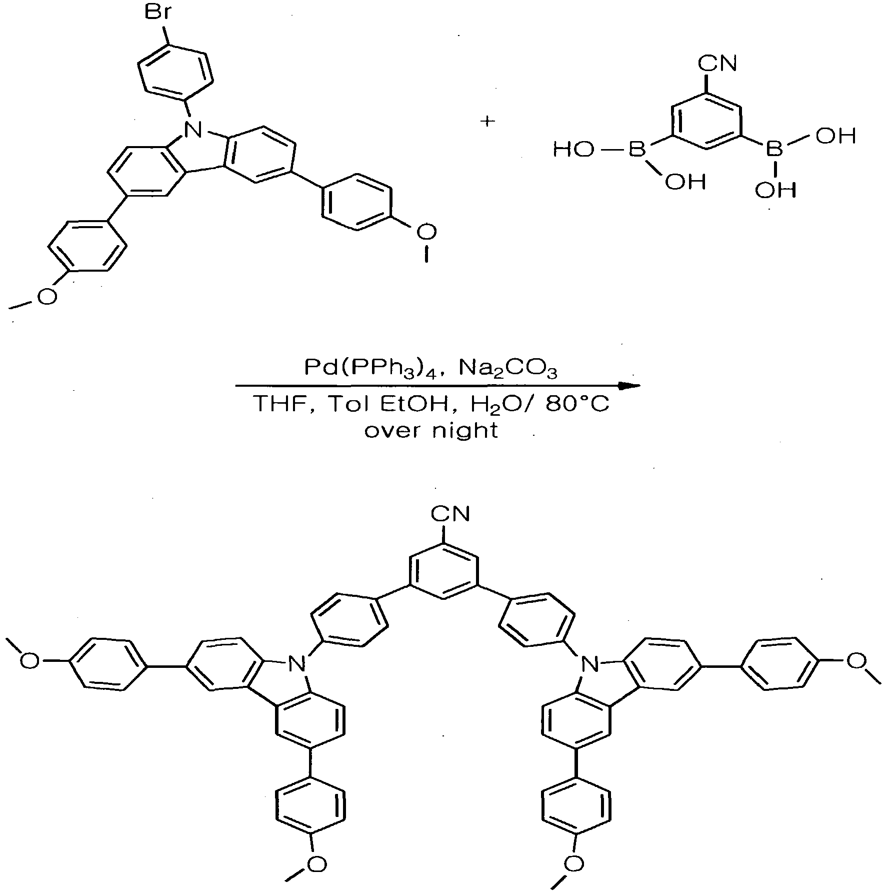

9-(4-bromophenyl)-3,6-bis(4-methoxyphenyl)-9H-carbazole (5.8 g, 11.01 mmol), 5-cyano-1,3-phenylenediboronic acid (2.90 g, 20.97 mmol), potassium carbonate (2.90 g, 20.97 mmol) and tetrakis(triphenylphosphine)palladium (0.61 g, 0.52 mmol) were dissolved in 100 mL of THF/toluene/ethanol/H₂O (bpb = 1:1:1:1) to prepare a mixed solution. After the reaction of the mixed solution at 80 □, the reacted mixed solution was extracted with water and ethyl acetate and dried by anhydrous magnesium sulfate and filtered, and then an organic solvent was volatilized, and a reaction product was separated by column chromatography (*n*-hexane/EtOAc = 2/1). The yield of the reaction product was 75% (4.1 g).
¹HNMR(300 MHz,CDCl₃) 8.21(d, -Ar, 4H), 7.5(s, -Ar, 3H), 7.25-7.29(d, -Ar, 8H), 3.8(s, CH₃-O, 12H), 7.02(d, -Ar, 4H), 7.65(d, -Ar, 4H), 7.4(d, -Ar, 4H), 7.2(d, - Ar, 4H)

### 8-8: Synthesis of 3,5-bis[4-(3,6-bis(4-methoxyphenyl)-9H-carbazole-9-yl)phenyl]benzoic acid

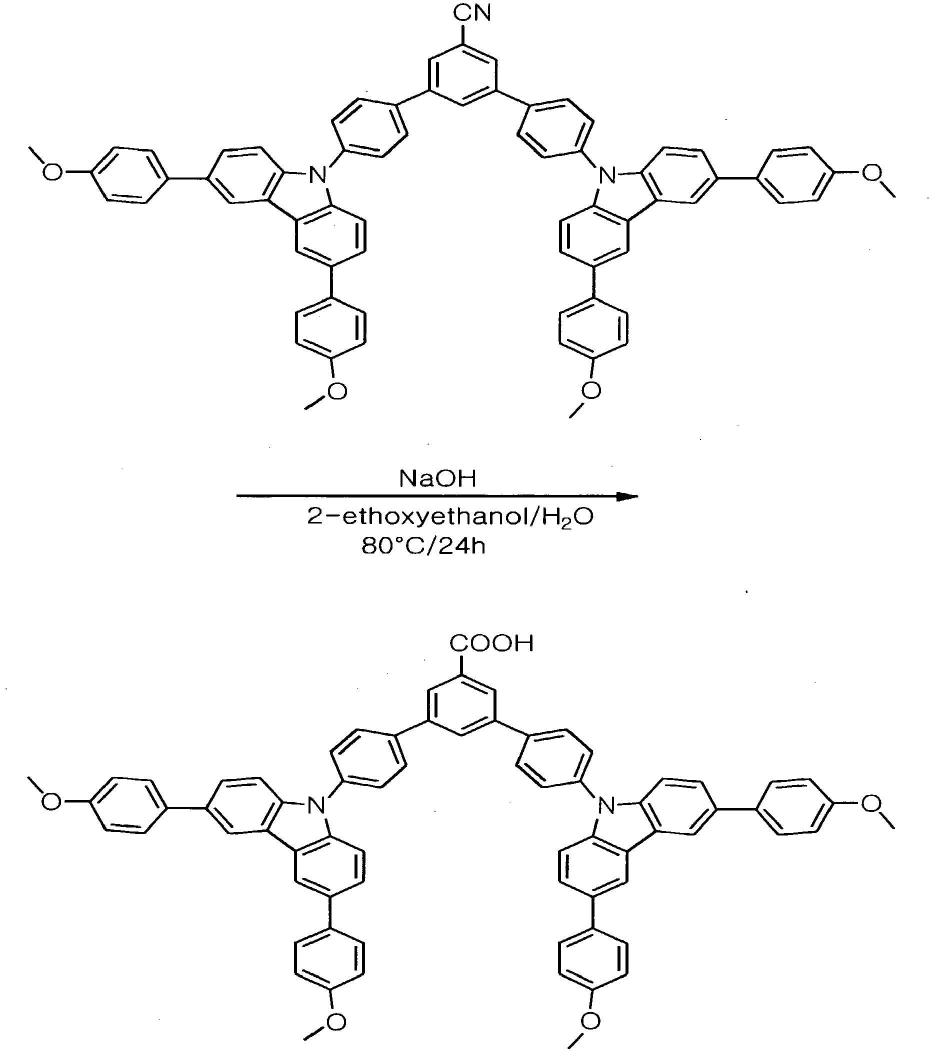

3,5-bis[4-(3,6-bis(4-methoxyphenyl)-9H-carbazole-9-yl)phenyl]benzonitrile (4.1 g, 4.06 mmol) and sodium hydroxide (0.32 g, 8.12 mmol) were dissolved in 20 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (n-hexane/EtOAc = 2/1). The yield of the reaction product was 94% (4.5 g).
¹HNMR(300 MHz,CDCl₃) 8.21(d, -Ar, 4H), 7.5(s, -Ar, 3H), 7.25-7.29(d, -Ar, 8H), 3.8(s, CH₃-O, 12H), 7.02(d, -Ar, 4H), 7.65(d, -Ar, 4H), 7.4(d, -Ar, 4H), 7.2(d, - Ar, 4H), 12.7(s, OH, H)

### Example 9: Synthesis of 4-(3,6-bis(2-ethylhexyloxy)-9H-carbazole-9-yl)benzoic acid

### 9-1: Synthesis of 4-(9H-carbazole-9-yl)benzonitrile

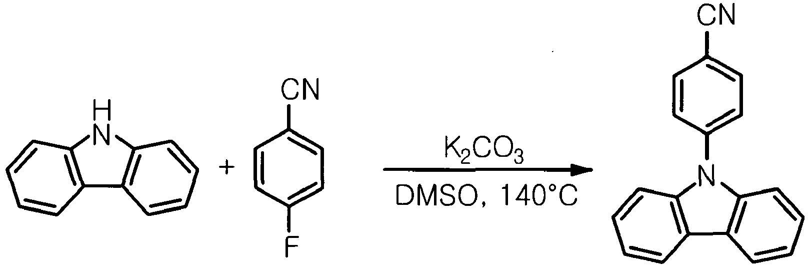

9H-carbazole (3 g, 17.94 mmol) was mixed with K₂CO₃ (8.22 g, 26.91 mmol) and DMSO (30 mL) and then stirred at room temperature for 1 hour to prepare a first mixture. Then, 4-fluorobenzonitrile (2.61 g, 21.5 mmol) was dripped into the first mixture and then stirred at 140°C for 12 hours to prepare a second mixture. After the reaction of the second mixture, a solvent was removed, the reaction mixture was extracted with water and ethyl acetate and dried by magnesium sulfate (MgSO₄), and then a reaction product was separated by column chromatography (*n*-hexane/EtOAc = 2/1). The yield of the reaction product was 94% (4.5 g).
¹HNMR(300 MHz,CDCl₃) 8.13(d, Ar-H, 2H), 7.44(d, Ar-H, 2H), 7.38(m, Ar-H, 2H), 7.32(d, Ar-H, 2H), 7.27(m, Ar-H, 2H), 7.10(d, Ar-H, 2H)

### 9-2: Synthesis of 4-(3,6-dibromo-9H-carbazole-9-yl)benzonitrile

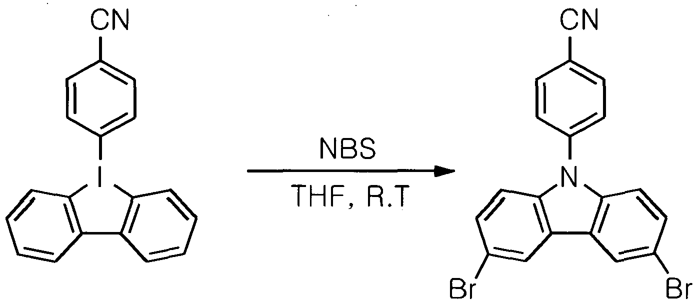

The obtained 4-(9H-carbazole-9-yl)benzonitrile (4.5 g, 16.8 mmol) was dissolved in THF (50 mL), and then *N*-bromosuccinimide (5.9 g, 33.9 mmol) was added thereto to prepare a mixed solution. Then, the mixed solution was reacted at room temperature for 4 hours. After the reaction of the mixed solution, a solvent was removed, and the reacted mixed solution was extracted with water and ethyl acetate to obtain an extract. The extract was dried by magnesium sulfate (MgSO₄), and then a reaction product was separated by column chromatography (*n*-hexane/EtOAc = 4/1). The yield of the reaction product was 90% (6.5 g).
¹HNMR(300 MHz,CDCl₃)δ7.01-7.03(d,-Ar,4H),7.48-7.51(d,-Ar,2H), 8.27(s,-Ar,2H)

### 9-3: Synthesis of 4-(3,6-bis(2-ethylhexyloxy)-9H-carbazole-9-yl)benzonitrile

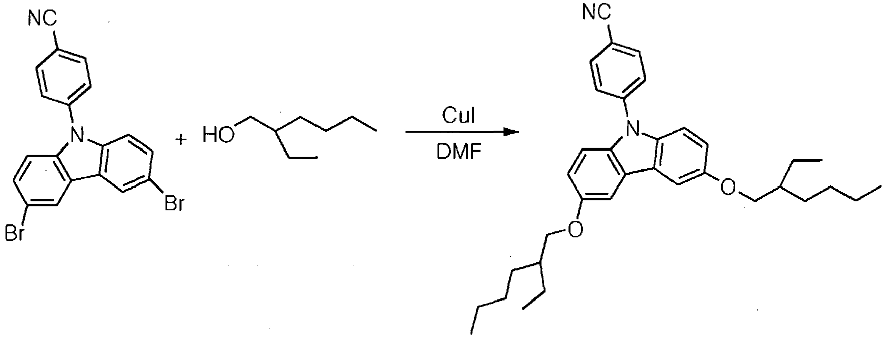

Sodium powder (1.5 g, 65.25 mmol) was mixed with 2-ethyl-1-hexanol (51.60 mL) and stirred for 1 day, and then 4-(3,6-dibromo-9H-carbazole-9-yl)benzonitrile (1.39 g, 3.26 mmol), CuI (2.61 g, 13.70 mmol) and DMF (30 mL) were added thereto and stirred for 12 hours to obtain an extract. The extract was dried by magnesium sulfate (MgSO₄), and then a reaction product was separated by column chromatography (*n*-hexane/ EtOAc = 4/1). The yield of the reaction product was 76% (1.3 g).
¹HNMR(300 MHz,CDCl₃)δ7.01-7.03(d,-Ar,4H),7.48-7.51(d,-Ar,2H), 8.27(s,-Ar,2H)
3.80-3.79(d, 2H, CH₂-O),1.73-1.67(m,1H,(CH2)3-H),1.53-1.28(m,8H,-CH₂),0.97-0.88(t,6H,-CH₃)

### 9-4: Synthesis of 4-(3,6-bis(2-ethylhexyloxy)-9H-carbazole-9-yl)benzoic acid

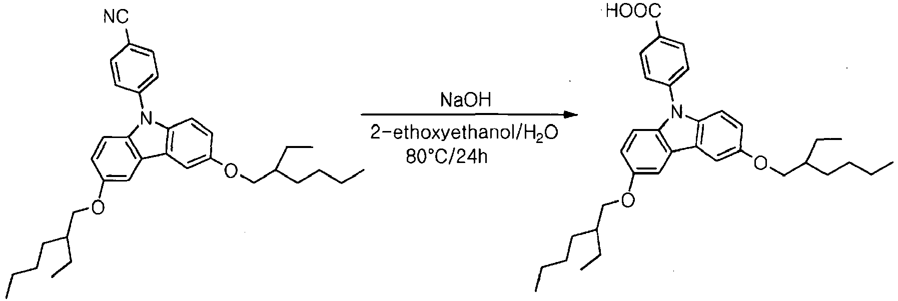

The obtained 4-(3,6-bis(2-ethylhexyloxy)-9H-carbazole-9-yl)benzonitrile (1.3 g, 2.47 mmol) and sodium hydroxide (0.51 g, 12.85 mmol) were dissolved in 20 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (n-hexane/EtOAc = 2/1). The yield of the reaction product was 94% (4.5 g).
¹HNMR(300 MHz,CDCl₃)δ7.01-7.03(d,-Ar,4H),7.48-7.51(d,-Ar,2H), 8.27(s,-Ar,2H)
3.80-3.79(d, 2H, CH₂-O), 1.73-1.67(m,1H,(CH₂)₃-H),1.53-1.28(m,8H,-CH₂),0.97-0.88(t,6H,-CH₃)

### Comparative Example 10: Synthesis of 4-(3,6-dihexyl-9H-carbazole-9-yl)benzoic acid

### 10-1: Synthesis of 3,6-diehexyl-9H-carbazole

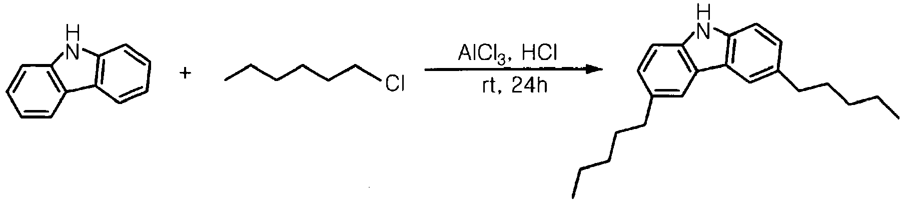

Carbazole (2.9 g, 17.4 mmol), 1-chlorohexane (9.65 g 134.2 mmol) and aluminum trichloride (2.32 g, 17.4 mmol) were mixed and then stirred for 1 hour to prepare a first mixture. Then, HCl (50 mL) was dripped into the first mixture and then stirred at room temperature for 24 hours to prepare a second mixture. After the reaction of the second mixture, the reaction mixture was extracted with water and ethyl acetate and dried by magnesium sulfate (MgSO₄), and then a reaction product was separated by column chromatography (*n*-hexane/EtOAc = 2/1). The yield of the reaction product was 24% (1.15 g).
¹HNMR(300 MHz,CDCl₃) 8.14(d, Ar-H, 2H), 7.46(dd, Ar-H, 2H), 7.34(d, Ar-H, 2H), 2.62-(m, 2H, Ar-CH₂),1.59-1.29(m,16H,-(CH₂)₃),0.97-0.88(m,6H,-CH₃).

### 10-2: Synthesis of 4-(3,6-dihexyl-9H-carbazole-9-yl)benzonitrile

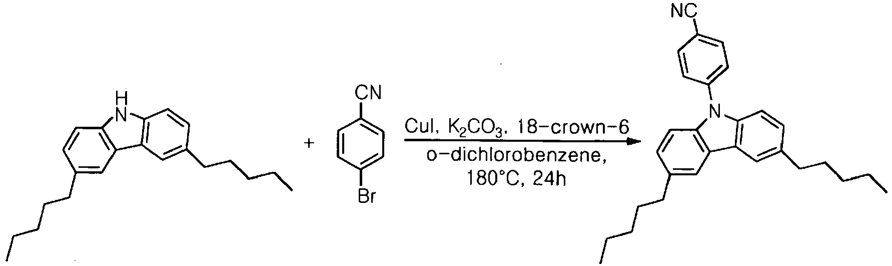

The obtained 3,6-dihexyl-9H-carbazole (1.15 g, 3.4 mmol) and 4-bromobenzonitrile (1.0 g, 5.49 mmol) were mixed with CuI (1.0 g, 5.49 mmol), 18-crown-6 (0.65 g, 2.46 mmol), K₂CO₃ (13.58 g, 98.25 mmol) and O-dichlorobenzene (30 mL) and then stirred at 180°C for 24 hours. After the reaction of the mixture, a solvent was removed, the reaction mixture was extracted with water and ethyl acetate and dried by magnesium sulfate (MgSO₄), and then a reaction product was separated by column chromatography (*n*-hexane/EtOAc = 2/1). The yield of the reaction product was 54% (1.3 g).
¹HNMR(300 MHz,CDCl₃) 8.14(d, Ar-H, 2H), 7.46(dd, Ar-H, 2H), 7.34(d, Ar-H, 2H), δ7.01-7.03(d,-Ar,4H), 2.62-(m, 2H, Ar-CH₂),1.59-1.29(m,16H,-(CH₂)₃),0.97-0.88(m,6H,-CH₃).

### 10-3: Synthesis of 4-(3,6-dihexyl-9H-carbazole-9-yl)benzoic acid

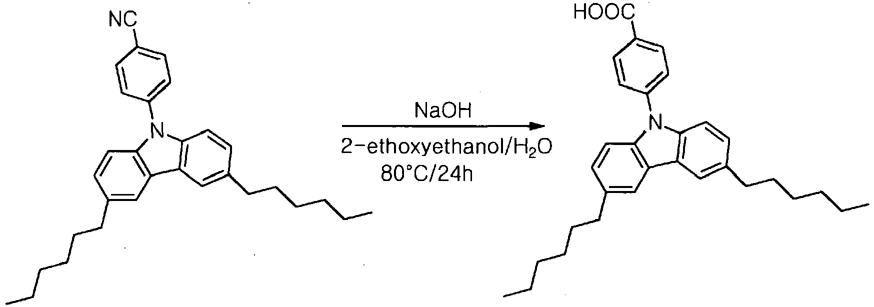

The obtained 4-(3,6-dihexyl-9H-carbazole-9-yl)benzonitrile (1.3 g, 2.6 mmol) and sodium hydroxide (1.51 g, 26.85 mmol) were dissolved in 40 mL of 2-ethoxyethanol/H₂O (2:1(v/v)), and then stirred at 80°C for 24 hours to prepare a mixed solution. After the reaction of the mixed solution, the reacted mixed solution was extracted with water and ethyl acetate and dried by magnesium sulfate (MgSO₄), and then a reaction product was separated by column chromatography (*n*-hexane/EtOAc = 2/1). The yield of the reaction product was 73% (3.0 g).
¹HNMR(300 MHz,CDCl₃) 8.14(d, Ar-H, 2H), 7.46(dd, Ar-H, 2H), 7.34(d, Ar-H, 2H), 7.13(d,-Ar,2H), δ7.01(d,-Ar,2H), 2.62-(m, 2H, Ar-CH₂),1.59-1.29(m,16H,-(CH₂)₃),0.97-0.88(m,6H,-CH₃).

### Comparative Example 11: Preparation of a dye solution containing a coadsorbent having hole conduction characteristics

A dye solution was prepared by dissolving the following dye and coadsorbent having hole conduction characteristics in ethanol such that the concentration of the dye and the concentration of the coadsorbent are given as follows.
NKX2677 dye [manufactured by Hayashibara Biochemical Lab., Inc., Japan]: 0.3 mmol (M)
*b*EHCBA [4-(3,6-bis(4-(2-ethylhexyloxy)phenyl)-9H-carbazole-9-yl)benzoic acid, Compound of Comparative Example 3]: 10 mmol (M)

### Examples 12 to 14: Preparation of dye solutions each containing a coadsorbent having hole conduction characteristics

Dye solutions each containing a coadsorbent having hole conduction characteristics were prepared in the same manner as in Example 11, except that 20 mmol (M) (Example 12), 30 mmol (M) (Example 13), and 40 mmol (M) (Example 14) of *b*EHCBAs [4-(3,6-bis(4-(2-ethylhexyloxy)phenyl)-9H-carbazole-9-yl)benzoic acids] were respectively dissolved with respect to 0.3 mmol (M) of a dye.

### Example 15: Preparation of a dye solution containing a coadsorbent having hole conduction characteristics

A dye solution was prepared by dissolving the following dye and coadsorbent having hole conduction characteristics in ethanol such that the concentration of the dye and the concentration of the coadsorbent are given as follows.
NKX2677 dye [manufactured by Hayashibara Biochemical Lab., Inc., Japan]: 0.3 mmol (M)
4-(N,N-bis(9,9-dimethyl-9H-fluorene-2-yl)amino)benzoic acid (compound of Comparative Example 2): 10 mmol (M)

### Comparative Example A: Preparation of a dye that does not contain coadsorbent having hole conduction characteristics

0.3 mmol (M) of NKX2677 was purchased and used as a dye.

### Comparative Example B: Preparation of a dye solution containing a dye and DCA (deoxycholic acid)

A dye solution was prepared by dissolving the following dye and DCA in ethanol such that the concentration of the dye and the concentration of the DCA are given as follows.
NKX2677 dye [manufactured by Hayashibara Biochemical Lab., Inc., Japan]: 0.3 mmol (M)
DCA: 40 mmol (M)

### Example 16: Manufacture of a dye-sensitized solar cell

A dye-sensitized solar cell was manufactured by the following steps.
1. An FTO glass substrate was immersed into a sodium hydroxide cleaning solution, washed with ultransonic waves, washed with distilled water and ethanol, and then dried using nitrogen gas.
2. The washed FTO glass substrate was immersed into a 40 mM aqueous TiCl₄ solution, and then heated in an oven at 70°C for 30 minutes.
3. The FTO glass substrate treated with TiCl₄ was washed with distilled water and ethanol, dried using nitrogen gas, and then heated in an oven at 80 °C for 10 minutes.
4. Subsequently, the FTO glass substrate treated with TiCl₄ was coated with TiO₂ paste having a particle size of 13 nm by a doctor blade method, and then dried at room temperature (20°C) for 2 hours.
5. The FTO glass substrate coated with TiO₂ was dried in an oven at 80°C for 2 hours.
6. Subsequently, the FTO glass substrate coated with TiO₂ was calcined at 500°C for 30 minutes while being slowly heated by a furnace.
7. The calcined FTO glass substrate was coated with TiO₂ paste having a particle size of 400 nm by a doctor blade method, dried at room temperature (20 °C) for 2 hours, and then calcined at 500°C for 30 minutes while being slowly heated by a furnace.
8. Subsequently, the calcined FTO glass substrate was immersed into a 40 mM aqueous TiCl₄ solution for 30 minutes, washed with distilled water and ethanol, dried using nitrogen gas, and then dried in an oven at 80°C for 10 minutes.
9. Subsequently, the dried FTO glass substrate was sintered for 30 minutes using a heating gun, and then immersed into the dye solution (EtOH) prepared in Example 11 to adsorb a dye and an adsorbent having hole conduction characteristics for 12 hours.
10. The FTO glass substrate adsorbed with the dye was washed with ethanol, and then dried using nitrogen gas.
11. Two inlets, each having a diameter of 0.6 mm, for injecting an electrolyte into the FTO glass substrate (counter electrode) were made.
12. Subsequently, the FTO glass substrate was immersed into an aqueous solution of H₂O/acetone/HCl (volume ratio = 4:4:2) for 1 hour, washed with ultrasonic waves, and then dried in an oven at 70°C for 30 minutes.
13. Subsequently, the FTO glass substrate was spin-coated with a Pt solution (prepared by dissolving 2 mg of H₂PtCl₆ in 1 mL of ethanol), and then heated at 400°C for 15 minutes using a heating gun.
14. The prepared oxidation electrode and reduction electrode were united by a polymer sealing film using a hot press heated to 80°C to form a cell.
15. An ionic electrolyte was injected into the cell using a vacuum pump line.
16. The inlet for injecting the ionic electrolyte into the cell was sealed with a polymer sealing film and cover glass.

### Examples 17 to 20: Manufacture of dye-sensitized solar cells

Dye-sensitized solar cells of Examples 17 to 20 were manufactured in the same manner as in Example 16, except that each of the dye solutions containing coadsorbents having hole conduction characteristics prepared in Example 12 (solar cell of Example 17), Example 13 (solar cell of Example 18), Example 14 (solar cell of Example 19) and Example 15 (solar cell of Example 20) was used instead of the dye solution containing a coadsorbent having hole conduction characteristics prepared in Example 11.

### Comparative Examples C and D: Manufacture of dye-sensitized solar cells

Dye-sensitized solar cells of Comparative Examples C and D were manufactured in the same manner as in Example 16, except that, in the step 9 of Example 16, each of the dye solutions containing coadsorbents having hole conduction characteristics prepared in Comparative Example A (solar cell of Comparative Example C) and Comparative Example B (solar cell of Comparative Example D) was used instead of the dye solution containing a coadsorbent having hole conduction characteristics prepared in Example 11.

### Test Example 1: Analysis of UV-vis absorption spectra of a dye containing a coadsorbent having hole conduction characteristics and adsorbed in a TiO₂ film, a dye containing no coadsorbent having hole conduction characteristics and DCA

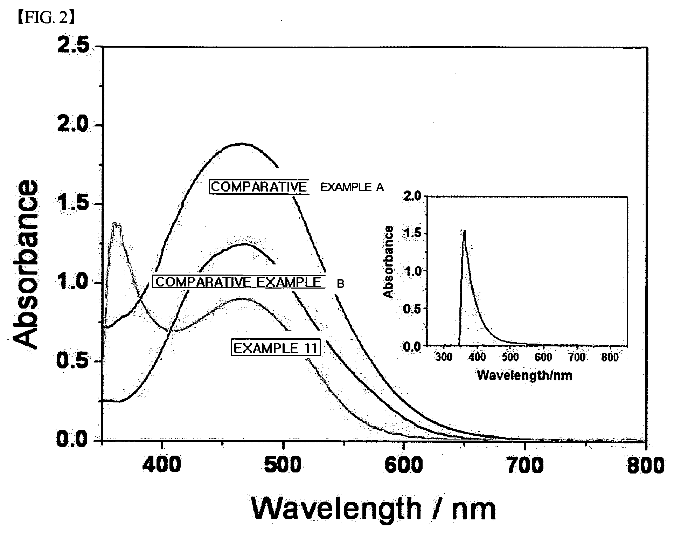

A TiO₂ film was dipped into each of the dye solutions prepared in Example 11, Comparative Example A and Comparative Example B for 12 hours to evaluate the absorbance thereof using UV-vis absorption spectra, and the results thereof are shown in FIG. 2.

### Test Example 2: Evaluation of performance of dye-sensitized solar cells

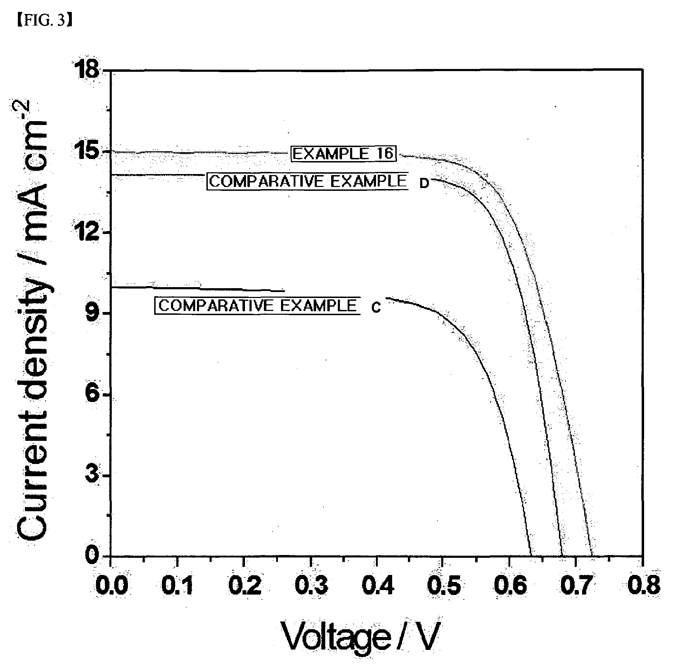

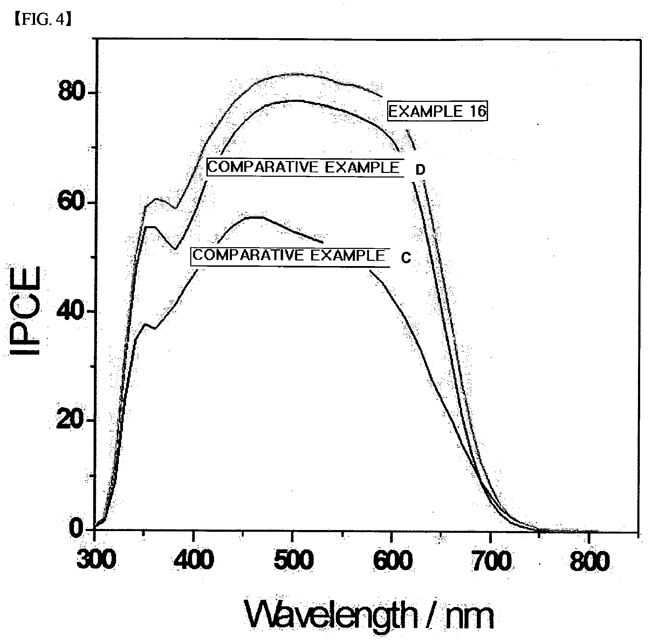

The photocurrent-voltage characteristics of the dye-sensitized solar cells manufactured in Example 16, Comparative Example C and Comparative Example D were measured under a condition of a 1 sun illumination of 100 mW/cm², and the results thereof are given in Table 1 below. The current-voltage curves of the dye-sensitized solar cells of Example 16, Comparative Example C and Comparative Example D are shown in FIG. 3, and the incident photon to current conversion efficiencies (IPCEs) thereof are shown in FIG. 4.

**[Table 1]**

| *Device* | *V_{oc}[V]* | *J_{sc}[mAcm⁻²]* | *FF [*%*]* | *η[%]* |
|---|---|---|---|---|
| Comp. Ex. C | 0.633 | 10.00 | 70.27 | 4.45 |
| Comp. Ex. D | 0.679 | 14.16 | 76.04 | 7.31 |
| Ex. 16 | 0.723 | 15.02 | 72.36 | 7.86 |

As shown in Table 1 and FIGS. 3 and 4, the dye-sensitized solar cell (Example 16) containing a coadsorbent (*b*EHCBA) having hole conduction characteristics exhibited *V*_{oc}: 0.723 V, *J*_{sc}: 15.02 mA/cm², Fill factor: 0.72.36% and *η:* 7.86% based on 1 sun (100 mW/cm²). The performance of the dye-sensitized solar cell (Example 16) was very remarkably improved compared to that of each of the dye-sensitized solar cells (Comparative Example C and Comparative Example D) containing no coadsorbent having hole conduction characteristics.

### Test Example 3: Internal charge transfer resistance characteristics of dye-sensitized solar cells

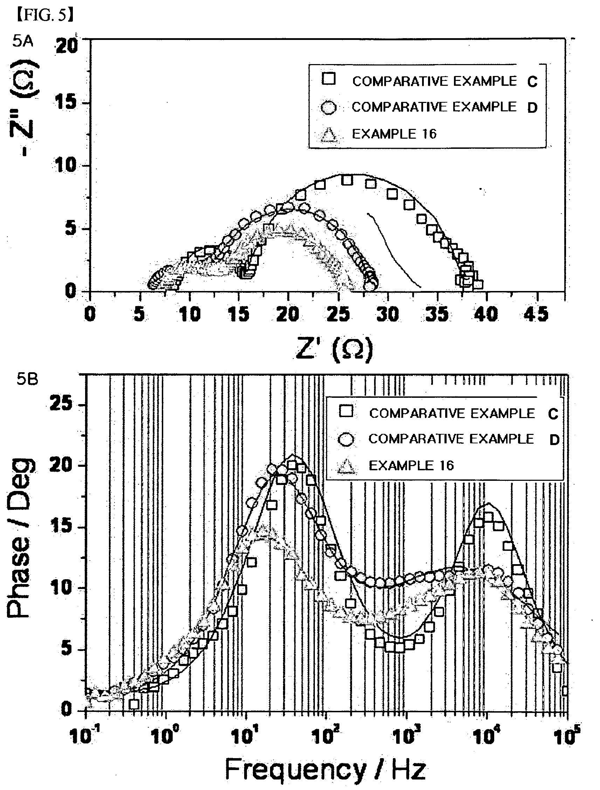

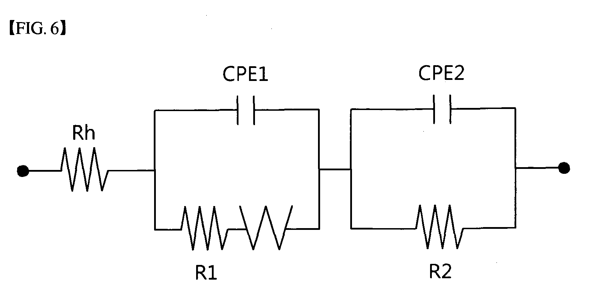

In order to evaluate the internal resistance characteristics of dye-sensitized solar cells and measure the charge transfer resistance therein, the Nyquist plot for fitting the AC impedances measured under a condition of 1 sun (100 mW/cm²) is shown in FIG. 5. In this case, an equivalent circuit set to obtain the internal resistance of each interface by impedance fitting is shown in FIG. 6.

In FIG. 6, Rh represents FTO resistance, R₁ represents TiO₂/dye/electrolyte interface resistance, R₂ represents Pt/electrolyte interface resistance, W represents Warburg impedance (resistance caused by mass transfer), and CPE represents capacitance (Q) caused by a constant phase element.

As shown in FIG. 5, the dye-sensitized solar cell (Example 16) containing a coadsorbent (*b*EHCBA) having hole conduction characteristics exhibited low resistance in the TiO₂/dye/electrolyte interface compared to that of each of the dye-sensitized solar cells (Comparative Example C and Comparative Example D). Such low resistance enables electrons to rapidly move, thus improving the efficiency of a dye-sensitized solar cell.

### Test Example 4: Evaluation of performance of dye-sensitized solar cells

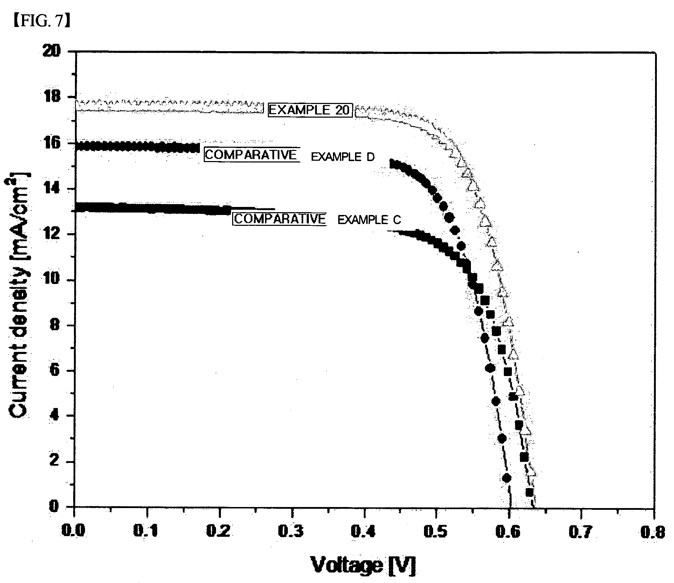

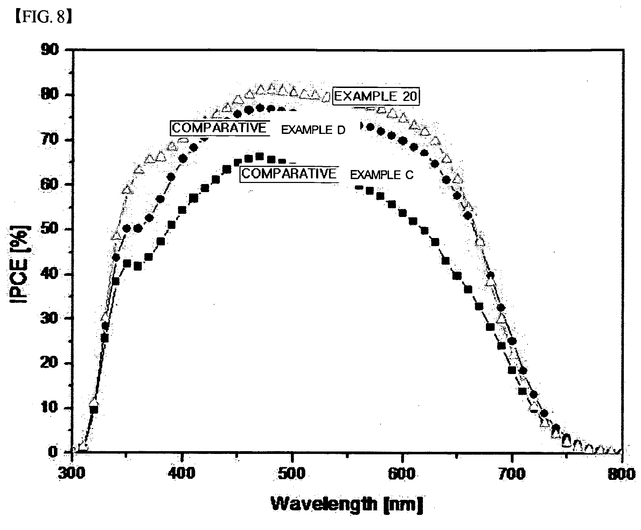

The photocurrent-voltage characteristics of the dye-sensitized solar cells manufactured in Example 20, Comparative Example C and Comparative Example D were measured under a condition of a 1 sun illumination of 100 mW/cm², and the results thereof are given in Table 2 below. The current-voltage curves of the dye-sensitized solar cells of Example 16, Comparative Example C and Comparative Example D were shown in FIG. 7, and the incident photon to current conversion efficiencies (IPCEs) thereof are shown in FIG. 8.

**[Table 2]**

| *Device* | *V_{oc}[V]* | *J_{sc}[mAcm⁻²]* | *FF [*%*]* | *η[%]* |
|---|---|---|---|---|
| Comp. Ex. C | 0.633 | 13.15 | 70.11 | 5.84 |
| Comp. Ex. D | 0.679 | 15.05 | 72.20 | 6.91 |
| Ex. 20 | 0.723 | 17.60 | 73.60 | 8.24 |

As shown in Table 2, the dye-sensitized solar cell (Example 20) containing a coadsorbent having hole conduction characteristics exhibited *V*_{oc}: 0.636 V, *J*_{sc}: 17.60 mA/cm², Fill factor: 73.60% and *η:* 8.24% based on 1 sun (100 mW/cm²). The performance of the dye-sensitized solar cell (Example 20) was very remarkably improved compared to that of each of the dye-sensitized solar cells (Comparative Example C and Comparative Example D) containing no coadsorbent having hole conduction characteristics.

## Claims

1. A compound, represented by Formula 3: 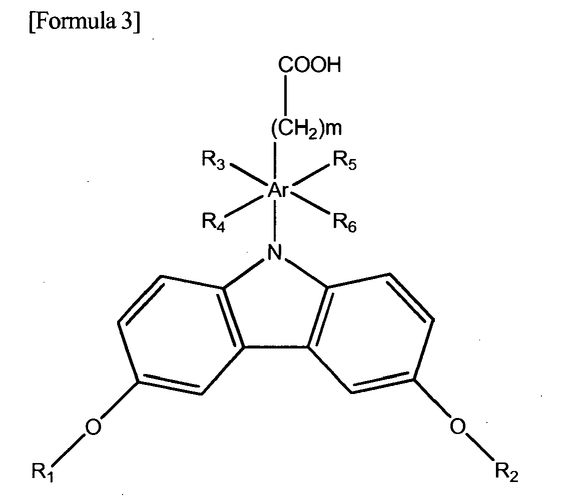 wherein Ar is an aromatic ring of C5 ~ C20 or an aromatic heterocycle of C5~C20, and the aromatic heterocycle includes 1 ~ 3 heteroatoms selected from the group consisting of O, S and N;
R1, R2, R3, R4, R5 and R6 are each independently hydrogen; alkyl of C1 ~ C15 unsubstituted or substituted with alkoxy of C1 ~ C15; alkoxy of C1 ~ C15 unsubstituted or substituted with alkyl of C1 ~ C15; alkoxy of C1 ~ C15 substituted with alkoxy of C1 ~ C15; aryl or heteroaryl of C5 ~ C20 unsubstituted or substituted with a substituent selected from the group consisting of alkyl of C1 ~ C15 unsubstituted or substituted with alkoxy of C1 ~ C15, alkoxy of C1 ~ C15 unsubstituted or substituted with alkyl of C1 ~ C15, and alkoxy of C1 ~ C15 substituted with alkoxy of C1 ~ C15; or arylalkyl or heteroarylalkyl of C6 ~ C22 unsubstituted or substituted with a substituent selected from the group consisting of alkyl of C1 ~ C15 unsubstituted or substituted with alkoxy of C1 ~ C15, alkoxy of C1 ~ C15 unsubstituted or substituted with alkyl of C1 ~ C15 and alkoxy of C1 ~ C15 substituted with alkoxy of C1 ~ C15;
m is an integer of 0 ~ 5; and
one or more of R3, R4, R5 and R6 are nonexistent when the Ar is an aromatic heterocycle.

2. A coadsorbent having hole conduction characteristics, comprising the compound of claim 1 represented by Formula 3.

3. A dye-sensitized solar cell, comprising a light absorbing layer including the coadsorbent of claim 2.

4. The dye-sensitized solar cell of claim 3, comprising:
a first electrode including a transparent conductive substrate;
a light absorbing layer formed on one side of the first electrode;
a second electrode facing the first electrode provided with the light absorbing layer; and
an electrolyte disposed between the first electrode and the second electrode.

## Patentansprüche

1. Verbindung, dargestellt durch Formel 3: 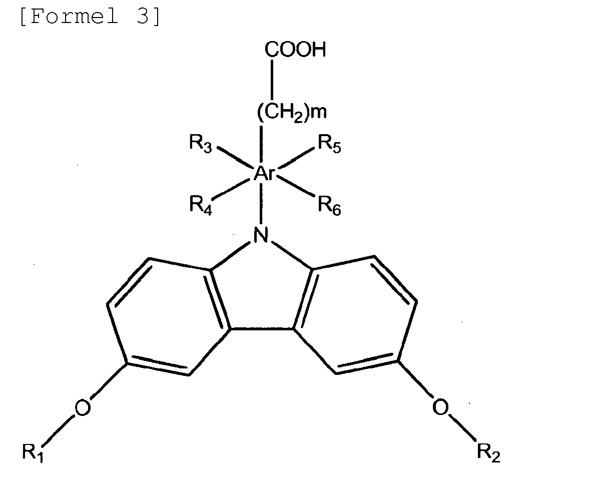 wobei Ar ein aromatischer Ring von C₅ bis C₂₀ oder ein aromatischer Heterocyclus von C₅ bis C₂₀ ist, und der aromatische Heterocyclus 1 bis 3 Heteroatome umfasst, die aus der Gruppe bestehend aus O, S und N ausgewählt sind;
R₁, R₂, R₃, R₄, R₅ und R₆ jeweils unabhängig Wasserstoff; Alkyl von C₁ bis C₁₅ unsubstituiert oder substituiert mit Alkoxy von C₁ bis C₁₅; Alkoxy von C₁ bis C₁₅ unsubstituiert oder substituiert mit Alkyl von C₁ bis C₁₅; Alkoxy von C₁ bis C₁₅ substituiert mit Alkoxy von C₁ bis C₁₅; Aryl oder Heteroaryl von C₅ bis C₂₀ unsubstituiert oder substituiert mit einem Substituenten ausgewählt aus der Gruppe bestehend aus Alkyl von C₁ bis C₁₅ unsubstituiert oder substituiert mit Alkoxy von C₁ bis C₁₅, Alkoxy von C₁ bis C₁₅ unsubstituiert oder substituiert mit Alkyl von C₁ bis C₁₅, und Alkoxy von C₁ bis C₁₅ substituiert mit Alkoxy von C₁ bis C₁₅; oder Arylalkyl oder Heteroarylalkyl von C₆ bis C₂₂ unsubstituiert oder substituiert mit einem Substituenten ausgewählt aus der Gruppe bestehend aus Alkyl von C₁ bis C₁₅ unsubstituiert oder substituiert mit Alkoxy von C₁ bis C₁₅, Alkoxy von C₁ bis C₁₅ unsubstituiert oder substituiert mit Alkyl von C₁ bis C₁₅ und Alkoxy von C₁ bis C₁₅ substituiert mit Alkoxy von C₁ bis C₁₅ sind;
m eine ganze Zahl von 0 bis 5 ist; und
eines oder mehrere von R₃, R₄, R₅ und R₆ nicht vorhanden sind, wenn das Ar ein aromatischer Heterocyclus ist.

2. Coadsorbens mit Löcherleiteigenschaften, umfassend die durch Formel 3 dargestellte Verbindung nach Anspruch 1.

3. Farbstoffsensibilisierte Solarzelle, umfassend eine lichtabsorbierende Schicht, die das Coadsorbens nach Anspruch 2 umfasst.

4. Farbstoffsensibilisierte Solarzelle nach Anspruch 3, umfassend:
eine erste Elektrode, die ein transparentes leitendes Substrat umfasst;
eine lichtabsorbierende Schicht, die auf einer Seite der ersten Elektrode ausgebildet ist;
eine zweite Elektrode gegenüber der ersten Elektrode, die mit der lichtabsorbierenden Schicht versehen ist; und
einen Elektrolyten, der zwischen der ersten Elektrode und der zweiten Elektrode angeordnet ist.

## Revendications

1. Composé représenté par la formule 3 : 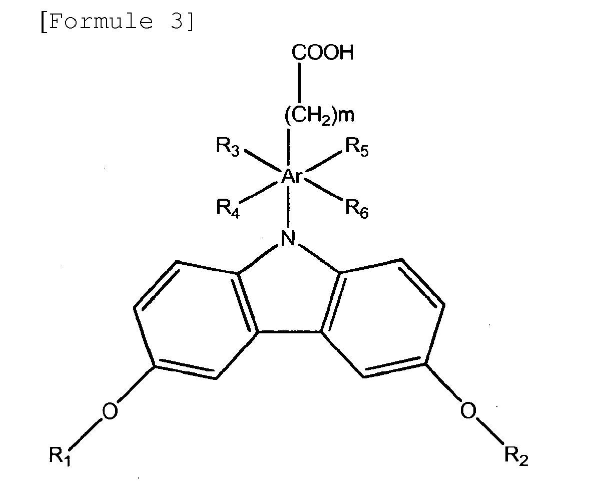 dans laquelle Ar est un cycle aromatique en C₅ à C₂₀ ou un hétérocycle aromatique en C₅ à C₂₀ et ledit hétérocycle aromatique comprend 1 à 3 hétéroatomes choisis dans le groupe constitué par O, S et N ;
R₁, R₂, R₃, R₄, R₅ et R₆ sont chacun, indépendamment, un atome d'hydrogène ; un groupe alkyle en C₁ à C₁₅ non substitué ou substitué par un groupe alcoxy en C₁ à C₁₅ ; un groupe alcoxy en C₁ à C₁₅ non substitué ou substitué par un groupe alkyle en C₁ à C₁₅ ; un groupe alcoxy en C₁ à C₁₅ substitué par un groupe alcoxy en C₁ à C₁₅ ; un groupe aryle ou hétéroaryle en C₅ à C₂₀ non substitué ou substitué par un substituant choisi dans le groupe constitué par un groupe alkyle en C₁ à C₁₅ non substitué ou substitué par un groupe alcoxy en C₁ à C₁₅, un groupe alcoxy en C₁ à C₁₅ non substitué ou substitué par un groupe alkyle en C₁ à C₁₅, et un groupe alcoxy en C₁ à C₁₅ substitué par un groupe alcoxy en C₁ à C₁₅ ; ou un groupe arylalkyle ou hétéroarylalkyle en C₆ à C₂₂ non substitué ou substitué par un substituant choisi dans le groupe constitué par un groupe alkyle en C₁ à C₁₅ non substitué ou substitué par un groupe alcoxy en C₁ à C₁₅, un groupe alcoxy en C₁ à C₁₅ non substitué ou substitué par un groupe alkyle en C₁ à C₁₅ et un groupe alcoxy en C₁ à C₁₅ substitué par un groupe alcoxy en C₁ à C₁₅ ;
m est un entier égal à un nombre de 0 à 5 ; et
un ou plusieurs parmi R₃, R₄, R₅ et R₆ n' existent pas quand Ar est un hétérocycle aromatique.

2. Co-adsorbant ayant des caractéristiques de transport de trous, comprenant le composé selon la revendication 1 représenté par la formule 3.

3. Cellule photovoltaïque sensibilisée au colorant, comprenant une couche absorbant la lumière comprenant le co-adsorbant selon la revendication 2.

4. Cellule photovoltaïque sensibilisée au colorant selon la revendication 3, comprenant :
une première électrode incluant un substrat conducteur transparent ;
une couche absorbant la lumière formée sur une face de la première électrode ;
une seconde électrode faisant face à la première électrode munie de la couche absorbant la lumière ; et
un électrolyte disposé entre la première électrode et la seconde électrode.
